# EUROPEAN PATENT SPECIFICATION

(11) **EP 1 254 251 B1**
(45) Date of publication and mention of the grant of the patent: **15.08.2007**
(21) Application number: 01903310.9
(22) Date of filing: 26.01.2001
(51) Int. Cl.: G01N 33/74

(54) **NEUROSTEROIDS AS MARKERS FOR ALZHEIMER'S DISEASE**
NEUROSTEROIDE ALS MARKER FÜR DIE ALZHEIMERSCHE KRANKHEIT
EMPLOI DE NEUROSTEROIDES COMME MARQUEURS POUR LA MALADIE D'ALZHEIMER

(30) Priority: 28.01.2000 US 178698 P
(43) Date of publication of application: 06.11.2002
(73) Proprietor: Georgetown University, Washington, DC 20057 (US)
(72) Inventor: PAPADOPOULOS, Vassilios, North Potomac, MD 20878 (US); BROWN, Rachel, C., Tucson, AZ 85719 (US); CASCIO, Caterina, I-90100 Palermo (IT)
(74) Representative: Daniels, Jeffrey Nicholas
(86) International application number: PCT/US2001/002476
(87) International publication number: WO 2001/055692

(56) References cited:
- US-A- 5 162 198
- CASCIO C ET AL: "Detection of P450c17-independent pathways for dehydroepiandrosterone (DHEA) biosynthesis in brain glial tumor cells" PROCEEDINGS OF THE NATIONAL ACADEMY OF SCIENCES OF THE UNITED STATES, vol. 95, no. 6, 17 March 1998 (1998-03-17), pages 2862-2867, XP002280552 March 17, 1998 ISSN: 0027-8424
- AISEN P S ET AL: "GLUCOCORTICOIDS IN ALZHEIMER'S DISEASE THE STORY SO FAR" DRUGS AND AGING, ADIS INTERNATIONAL LTD, NZ, vol. 12, no. 1, January 1998 (1998-01), pages 1-6, XP001079609 ISSN: 1170-229X
- DATABASE BIOSIS [Online] BIOSCIENCES INFORMATION SERVICE, PHILADELPHIA, PA, US; 1996 YANASE TOSHIHIKO ET AL: "Serum dehydroepiandrosterone (DHEA) and DHEA-sulfate (DHEA-S) in Alzheimer's disease and in cerebrovascular dementia" Database accession no. PREV199698779408 XP002280554 & ENDOCRINE JOURNAL, vol. 43, no. 1, 1996, pages 119-123, ISSN: 0918-8959
- BROWN RACHEL C ET AL: "Oxidative stress-mediated DHEA formation in Alzheimer's disease pathology." NEUROBIOLOGY OF AGING, vol. 24, no. 1, 2003, pages 57-65, XP002280553 ISSN: 0197-4580
- PRASAD ET AL.: 'Precursors fo the neurosteroids' PROC. NATL. ACAD. SCI. USA vol. 91, 1994, pages 3220 - 3223, XP002946898
- BASTIANETTO ET AL.: 'Dehydroepiandrosterone (DHEA) protects hippocampal cells from oxidative stress-induced damage' MOLECULAR BRAIN RESEARCH vol. 66, 1999, pages 35 - 41, XP002946899

## Description

### Field of Invention

The present invention concerns diagnostic methods for detecting neuropathologies, and particularly Alzheimer's disease, by testing for increased levels of central nervous system dehydroepiandrosterone (DHEA). In particular, the present inventors have found that DHEA in the brains of patients with neuropathologic disease is formed by an alternative metabolic pathway than that used for the synthesis of peripheral DHEA, and that a concomitant decrease in a steroid precursor involved in this alternative pathway may also serve as an indicator of neuropathology. Understanding the mechanisms by which neuroactive steroids are synthesized within the human brain and the roles of neurosteroids in normal and pathological brain function will also enable the design of better targeted drug treatments for neuropathologic disorders.

### Background of the Invention

Aisen and Pasinetti (Drugs & Aging, vol 12, no- 1, 1998, pages 1-6) reviewed glucocorticoids in Alzheimer's Disease.

Bastianetto et al., (Molecular Brain Research, vol 66, issue 1-2, 20 March 1999, pages 35-41) investigated the antioxidant effects of DHEA in models of oxidative stress using rat primary hippocampal cells and human hippocampal tissue from Alzheimer's Disease patients and age-matched controls.

Toshihiko et al., (Database Biosis, Accession No. PREV199698779408) have submitted an abstract to the Endocrine Journal titled "Serum dehydroepiandrosterone (DHEA) and DHEA-sulfate (DHEA-S) in Alzheimer's disease and in cerebrovascular dementia".

Dehydroepiandrosterone (DHEA) is a major adrenal steroid in humans (1). Peripheral levels peak early in adulthood and gradually decline with aging (1, 2). Its role in the periphery is not well established, but it is thought to serve mainly as a precursor for androgens (3). It has been demonstrated that levels of DHEA in the brain exceed those seen in the periphery, and these levels are maintained after the removal of peripheral steroidogenic organs (4, 5). DHEA and its sulfated form are considered neuroactive in that they have effects on the GABAA receptor complex and NMDA-mediated glutamate transmission via sigma receptors (6, 7). Furthermore, DHEA and DHEA sulfate (DHEA-S) are thought to be involved in modulating learning and memory (8, 9).

The role of DHEA as a neurosteroid is well established (10). However, the mechanism by which DHEA is produced in the brain has been the source of some debate. Evidence for the existence of active cytochrome P450 17α-hydroxylase (P450c17), the peripheral steroidogenic enzyme that converts pregnenolone to DHEA, has been contradictory. There is some evidence for the expression of mRNA for P450c17 early in development (11), but the same group that reported this finding was not able to detect mRNA in the adult rodent (12). One report has indicated expression in the adult rodent (13), but evidence for either protein or enzymatic activity has not been forthcoming (14, 5).

Neurosteroids have been implicated in a number of different clinical conditions including memory (9, 26, 85), depression (84), and pre-menstrual syndrome (67). They have also been proposed to be potential drugs for the treatment of anxiety (36, 37, 40, 46) and epilepsy (35, 41, 47) via their ability to potentiate GABA inhibition (65). Although several studies have looked at the regional distribution of neurosteroids in the human brain (30, 31, 38), there is no evidence as to where these steroids were synthesized. In addition, there have been no published studies examining the mechanisms of regulation of neurosteroid biosynthesis in either rodent or human brain, assuming it occurs.

Despite the lack of information regarding the role of neurosteroids in the brain and the mechanisms of brain cell-derived neurosteroid synthesis, there has been much speculation on the role of DHEA, or the declining levels thereof, in the aging process (1, 2). Because peripheral DHEA decreases with age in the human, it has been assumed that DHEA also decreases with aging in the brain. However, there are no valid studies available to back up this hypothesis. A number of studies have tried to correlate peripheral levels of DHEA and DHEA-S with cognitive function (23-25) or with future development of dementia or Alzheimer's disease (26). In fact, one internet website currently advertising a DHEA supplement reports that DHEA-S levels are significantly lower in patients with early or late Alzheimer's disease compared to normal controls. Although there have been many proclamations in relation to DHEA supplements regarding potential advantages for improving memory and cognitive functions, scientific studies have been unable to demonstrate conclusively a connection between peripheral DHEA levels and cognitive ability, or to use peripheral DHEA levels as a predictor of future neuropathology.

It would be advantageous to further the understanding of neurosteroid synthesis and regulation in the brain for the purpose of developing a better understanding of brain diseases and the aging process, and for the potential this knowledge would create for the design of new drugs and diagnostic assays.

### Summary of the Invention

In contrast to what has been reported in the literature, the present inventors have surprisingly found that central nervous system (CNS) DHEA is increased in patients having Alzheimer's disease. This finding contradicts previous reports that allege that DHBA levels in Alzheimer's patients, including those in the brain, are abnormally low, and has significant relevance given the fact that many companies are currently advocating DEBA supplements as a means to prevent the development of Alzheimer's disease. Moreover, the present inventors have also found that, contrary to the focus of prior studies, DHEA is synthesized in the brain via an alternative metabolic pathway than that used in the periphery. These findings underlie the methods of the present invention, the aim of which is to diagnose or detect neuropathologic diseases, and in particular, Alzheimer's disease.

The methods of the invention involve measuring the amounts of neurosteroids in the central nervous system and serum of patients. In particular, the methods involve screening for increased levels of DHEA, or for decreased levels of newly identified precursor for DHEA, as indicators of the early onset of neurodegenerative disease. Methods of making or increasing the synthesis of DHEA by exposing cells to β-amyloid protein are also included, given the finding reported herein that β-amyloid protein and other agents or conditions inducive of oxidative stress may have a regulatory role in the alternative pathway for DHEA synthesis. Understanding mechanisms of neurosteroid biosynthesis and regulation thereof in the brain, and deciphering the nature of the alternative metabolic pathway for brain DHEA and the role of increased DHEA production in neuropathologic disease, will permit the design of proactive treatments geared toward preventing or treating disease progression in patients identified by the disclosed screening methods.

### Brief Description of the Drawings

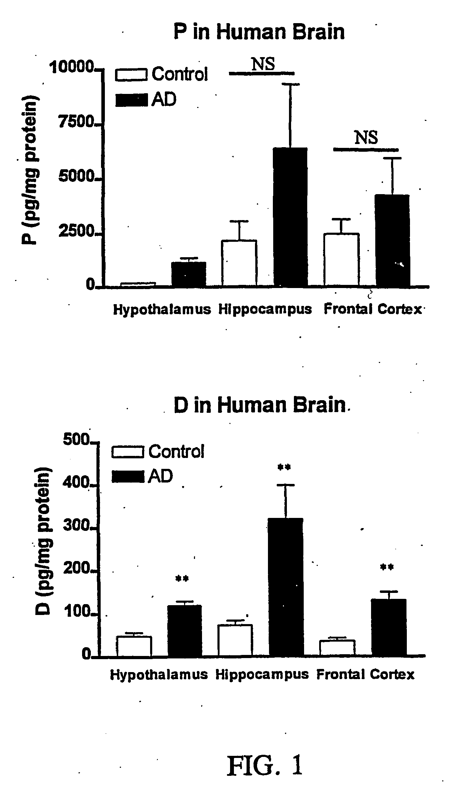

Figure 1: Neurosteroid Levels in Alzheimer's Disease (AD) and Control Brain
   Endogenous neurosteroid levels in human brain were measured by specific radioimmunoassay after HPLC purification. There is no significant difference in pregnenolone levels between AD and control patients. However, all three regions examined show a significantly higher level of DHEA in AD patients. Data presented is means ±SEM for duplicate measurements from 3-10 samples. NS - not significant, **p<0.01.

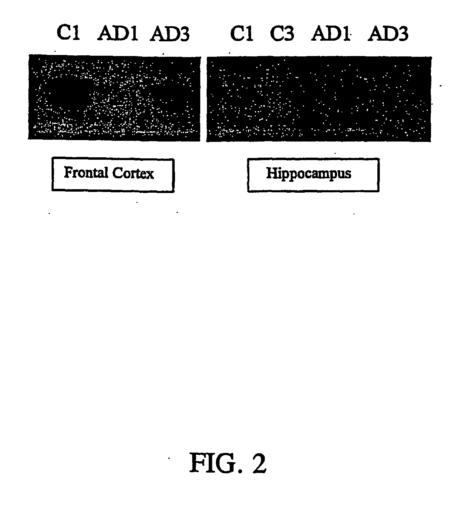

Figure 2: mRNA Expression of P450c17 in AD and Control Brain
   RT-PCR amplification of P450c17 followed by DNA blot indicates that P450c17 is expressed in the cortex of both control and AD patients, although expression is only seen in one of the available AD patient samples. There is no detectable expression of P450cl7 in the hippocampus, indicating that DHEA in this area is not made via the peripheral enzyme-mediated pathway. Lanes are labeled by patient number to allow for within patient comparisons.

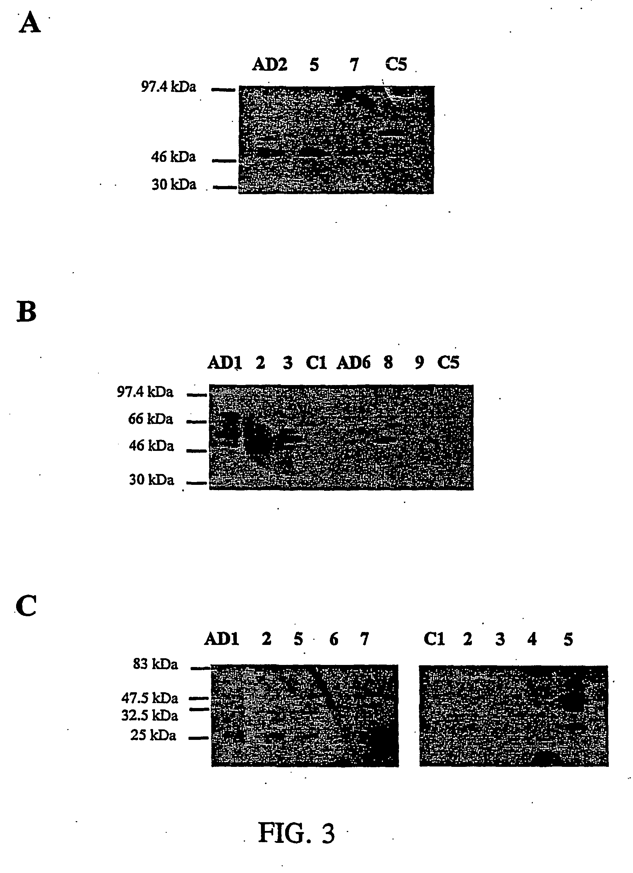

Figure 3: Aβ Immunoreactivity in AD and Control Brain
   Brain homogenates were separated by SDS-PAGE and blotted with 4G8, a monoclonal antibody to amino acids 17-24 of the β-amyloid (Aβ) peptide. A) The majority of Aβ-immunoreactivity in the hypothalamus of AD patients is of a lower molecular weight species than in control patients. There is detectable Aβ in the control sample as well. B) Aβ-immunoreactivity is undetectable in the hippocampus of control patients, but is present in the hippocampus of AD patients. C) Aβ-immunoreactivity is present in frontal cortex from all AD patients with a similar pattern of molecular weight species. The majority of the AD frontal cortex Aβ-immunoreactivity is about 30 kDa. There is some Aβ-immunoreactivity in control patients, particularly in patient C5, but this immunoreactivity is at a higher molecular weight, about 50 kDa, then in AD patients. Lanes are labeled by patient number to allow for within patient comparisons.

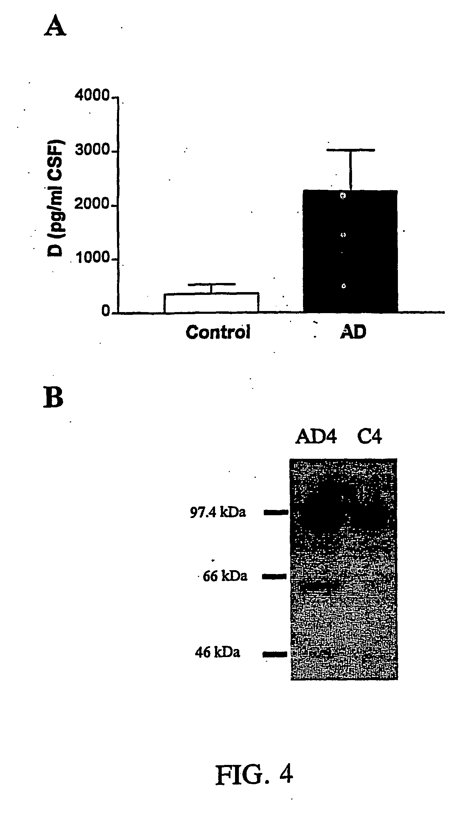

Figure 4: DHEA and Aβ Levels in Cerebrospinal Fluid (CSF) from AD and Control Patients
   A) DHEA was purified from CSF extracts by HPLC and measured by specific radioimmunoassay. DHEA levels are much higher in AD subjects then in control. B) Both AD and control patients have immunoreactive Aβ in the CSF. Control patients have only a 98 kDa species, while AD patients have AP-immunoreactivity occurring at a wide range of molecular weights from 46-98 kDa.

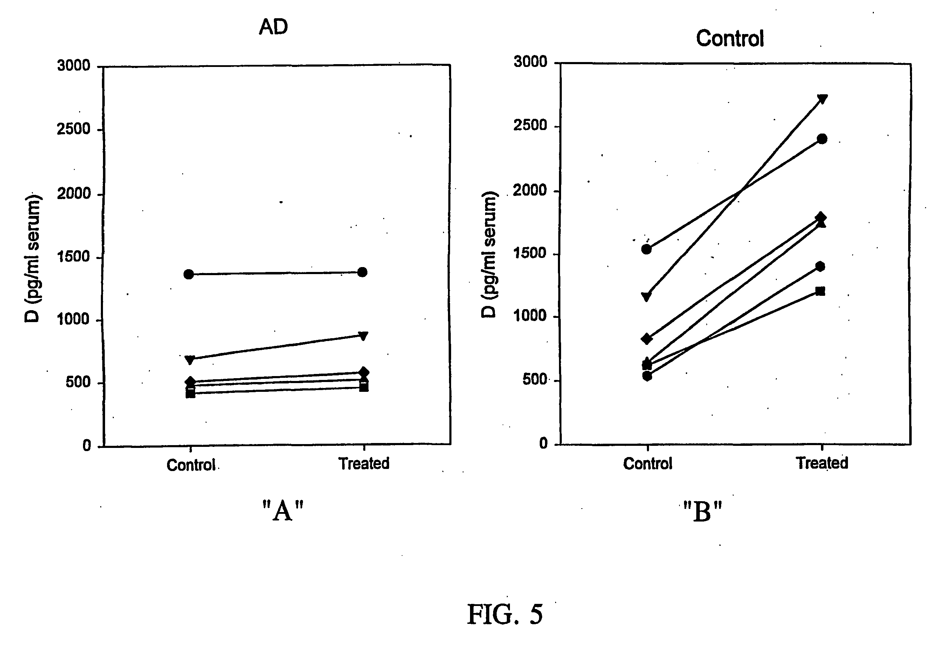

Figure 5: Serum Response to FeSO4 in AD and Control Patients
   When serum from AD patients (A) is treated with 10 mM FeSO₄, there is no increase in DHEA levels. However, when serum from control patients (B) is treated with 10 mM FeSO₄, there is a large induction in DHEA levels, indicating the presence of a DHEA precursor that can be acted upon by oxidizing agents. AD patients do not have this precursor present, potentially because it has already been converted in the brain before reaching the periphery.

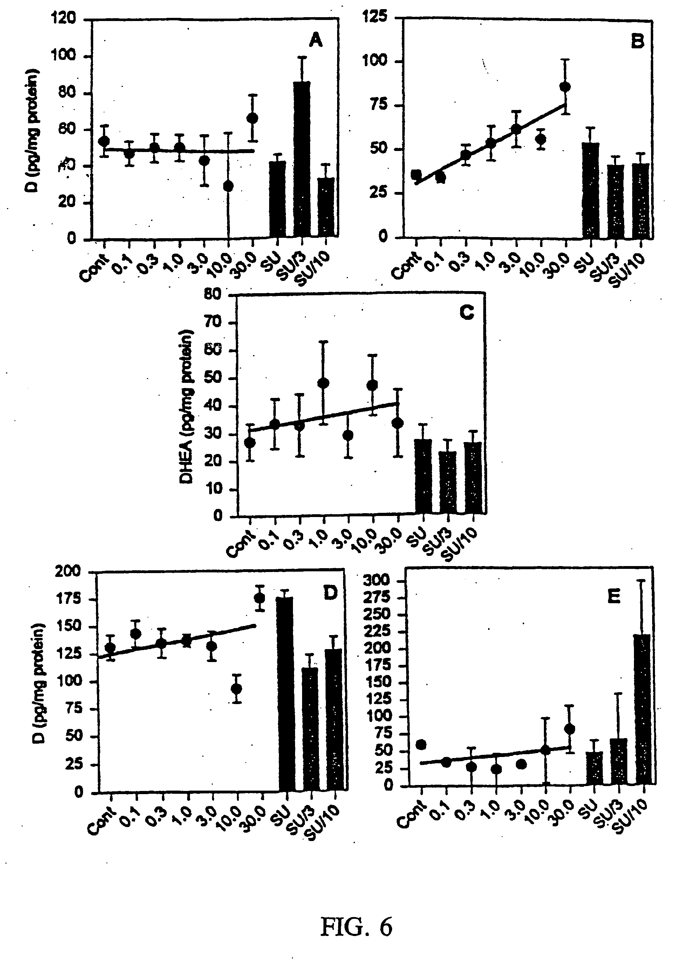

Figure 6: Human brain cells have an alternative pathway for D synthesis.
   Cells were treated with increasing concentrations of FeSO₄ (mM), with and without 5 mM SU 10603. After two hours, cells and media were processed as extracted with ether:ethyl acetate, samples were run on reverse-phase C18 TLC plates, fractions were extracted with diethyl ether and quantities of D were determined by RIA. D levels (pg/mg protein) were analyzed by one-way ANOVA. (A) KG-1-C; F=1.948, p=0.0979 (B) MGM-1; F = 5.004, p < 0.001 (C) MGM-3; F = 0.5902, p = 0.7366 (D) NHA; F = 4.131, p < 0.01 and (E) hNT-PF; F = 0.6552, p = 0.6886. Data is shown as means SEM (n = 4-15). All experiment were perfomed in the absence of exogenous P except for the hNT-PF cells, which were incubated with 5 mM P in addition to FeSO₄. Labels on X axis indicate mM dose of FeSO₄; SU, SU/3 and SU/10 indicate the addition of 5 mM SU 10603 along with the corresponding mM dose of FeSO₄.
Figure 7: FeSO₄ increases levels of ROS in MGM-1 and NHA cells.
   FeSO4 treatment causes a significant increase in MGM-1 and NHA cellular ROS levels at all doses tested (•, MGM-1: F = 59.883, p < 0.0001; ■, NHA: F = 25.419, p < 0.0001). NHA cells are more sensitive to Fe²⁺ in terms of ROS production then MGM-1 human glioma cells.
Figure 8: β-Amyloid increases cellular ROS and D synthesis.
   MGM-1 cells were treated with β-amyloid (Aβ) alone or with Aβ and Vitamin E for at least 24 hours. Cellular ROS and D levels were measured. Aβ caused a significant increase in ROS above control levels (** p < 0.01) and this could be partially blocked by co-treatment with Vitamin E (p = 0.054 versus Ab) but was still significantly above control levels (** p < 0.01). Similarly, Ab significantly increased D levels above control (** p < 0.01), and this effect was blocked by Vitamin E (* p < 0.05 versus Ab). Data is shown as mean increase above control SEM (n =12).

### Detailed Description of the Invention

The present inventors have found that patients in the early stages of oxidative stress-related neuropathological diseases can be identified by virtue of increased levels of DHEA in the brain, and that the DHEA in these patients is synthesized in the brain by an alternative metabolic pathway (16). This pathway may be regulated by intracellular free radicals and reactive oxygen species through a novel precursor, as the absence or decrease of this precursor parallels the observed increase in DHEA in the CNS of these patients. Although the precise identity of the precursor is not yet known, its presence can be measured by the addition of FeSO₄ and other reducing agents which presumedly cause a classical Fenton reaction and formation of DHEA if the precursor is present (15).

The generation of oxygen-free radicals by the addition of Fe²⁺ to cells has been previously described (88, 89). Generation of oxygen free radicals has been implicated in neurodegeneration (44), and evidence of oxidative stress has been shown in Alzheimer's disease brain (20, 21, 29), where oxidative stress contributes to the formation of amyloid plaques and neurofibrillary tangles (45, 83). β-amyloid is a component of Alzheimer's disease plaques (18) and can cause increases in reactive oxygen species (ROS) via several mechanisms (55, 58, 59).

Considering the effect of FeSO₄ on DHEA formation and the effects of Fe²⁺ and β-amyloid on ROS, and given the discovery reported herein that DHEA levels are increased in the brains of Alzheimer's patients, the present inventors hypothesized that the alternative metabolic pathway for DHEA synthesis might be stimulated by β-amyloid. Consequently, when oligodendrocytes were exposed to β-amyloid, these cells exhibited increased DHEA synthesis. The effects of β-amyloid were in turn blocked by vitamin E, which is known to be a powerful anti-oxidant (52). The observation that DHEA levels are elevated in the brains of Alzheimer's and the observed role of β-amyloid on DHEA synthesis by oligodendrocytes together suggest that elevated DHEA levels may be employed as an early indication of neuropathological processes.

Accordingly, the present invention includes a method of detecting oxidative stress-mediated Dehydroepiandrosterone (DHEA) formation, ie., formation of DHEA by the alternative metabolic pathway, in a test sample of blood, body fluid or tissue by screening for the absence of or a decrease in the normal levels of precursor. Although the identity of the precursor is still unknown, its presence can be determined by treating cells with an agent that causes the formation of ROS i.e. a β-amyloid peptide and screening for the subsequent formation of DHEA. Moreover, although this phenomenon had been reported prior to the present invention, the fact that this precursor would be absent or decreased in any particular tissue as to justify a comparative screening assay was not revealed until the present invention.

Thus, one diagnostic method according to the present invention includes a method of detecting oxidative stress-mediated DHEA formation in a test sample of blood, body fluid or tissue comprising:
(a) treating said test fluid or tissue with an agent that produces oxidative metabolites in a control fluid or tissue,
(b) treating a control fluid or tissue with said agent, and
(c) determining whether the quantities of DHEA in the test sample and in the control sample rise in response to said treatment,
wherein said agent is a β-amyloid peptide, and whereby a lack of response in the test sample relative to the control sample indicates that oxidative stress-mediated DHEA formation occurred previously in said test sample of body fluid.

"Oxidative stress-mediated DHEA formation" refers particularly to DHEA formed by an alternative metabolic pathway, which differs from the classical synthesis pathway used in the periphery that purportedly relies on cytochrome P450 17α-hydroxylase (P450c17) to convert pregnenolone to DHEA (11). Although the present inventors do not wish to be held to any specific theory, the alternative pathway may involve hydroperoxide intermediates, and particularly a C-17 or C-20 oxygenated steroid (28). Preliminary data using CSF from schizophrenic patients, a disease independent of oxidative stress, shows DHEA levels similar to control patients, further supporting the validity of the hypothesis that the alternative metabolic pathway may be particularly relevant to neuropatholigies involving the formation of reactive oxygen species (ROS). The fact that the elevated levels of DHEA seen in neuropathologies involving oxidative stress specifically parallel decreases in an ROS-regulated precursor is a significant showing, given that, prior to the present invention, there was no information available about the synthesis and regulation of neurosteroids in the brain.

After examining the ability of various types of brain cells to synthesize DHEA via the alternative pathway, the present inventors have found that oligodendrocytes and astrocytes can make DHEA via this pathway, but neurons do not. Thus, a patient in the beginning stages of a brain disorder might be diagnosed by showing decreased levels of precursor in a sample of oligodendrocytes or astrocytes according to the method described above.

Oligodendricyte cells and astrocytes can be identified by the presence of markers specific to that cell type. For instance, both MBP (myelin baisc protein) and 2',3'-cyclic nucleotide-3'-phosphodiesterase are oligodendrocyte-specific markers, whereas glial fibrillary acidic protein (GFAP) is an astrocyte-specific marker. Other markers specific for these cell types are known in the art, and could be readily used by one of skill in the art to verify the identity of cells employed in the present assay, for instance using RT-PCR or other screening techniques.

Various cell lines have been characterized herein by virtue of their ability to synthesize DHEA via the alternative metabolic pathway, and these cell lines might be used as a convenient source of control cells for the assay. For instance, FeSO₄ has a significant effect on DHEA formation by NHA cells, an astrocyte cell line. Thus, this cell line could be used as a positive control, according to the methods described herein. MGM-1, derived from a glioblastoma multiforme tumor (70), also responds to FeSO₄ treatment, as well as to treatment with β-amyloid, by increased ROS formation and DHEA synthesis. In contrast, neither MGM-3 cells (70) nor KG-1-C cells (69) respond to FeSO₄ treatment; and might be employed as negative controls.

The inventors have further found that FeSO₄ treatment results in a significant increase in DHEA in the hippocampus and cortex of control patients, indicating the presence of an alternative precursor in these patients. There was no effect observed in the hypothalamus of control patients. In contrast, Alzheimer's patients showed a significant increase in DHEA levels in response to FeSO₄ treatment only in the frontal cortex, but not in the hippocampus or hypothalamus. Given the presence of higher levels of DHEA in the Alzheimer's patient brain before FeSO₄ and the difference in increase in DHEA levels in the hippocampus in Alzheimer's patients versus controls patients following FeSO₄ treatment, this suggests that the precursor in the hippocampus of Alzheimer's patients has already been converted to DHEA. Thus, a patient in the beginning stages of a brain disorder might be diagnosed by showing decreased levels of precursor in a sample of hippocampus tissue according to the method described above.

The diagnostic method disclosed above might be most readily performed, however, with cerebrospinal fluid (CSF) or serum. Both of these bodily fluids exhibit decreased formation of DHEA in response to FeSO₄ treatment when taken from Alzheimer's patients versus control patients. The fact that CSF fluid may be used as a diagnostic tool in the assays of the invention is perhaps logical given the inventors' other findings about oxidative stress-induced DHEA formation in the Alzheimer's disease brain. The finding that serum, however, can be used in the above method as a tool for diagnosing oxidative stress-induced formation of DHEA in the brain was somewhat surprising in this context. For instance, the DHEA levels in the serum of Alzheimer's disease patients are not significantly greater than the serum levels in control patients. Therefore; the fact that the precursor levels in the serum of Alzheimer's patients are nevertheless decreased suggests that the precursor that is normally found in serum has already been metabolized in the brains of these patients. Thus, the first diagnostic method introduced above may be performed with a variety of body fluids and tissue samples, wherein said samples are selected from the group consisting of cerebrospinal fluid, serum, hippocampus tissue, oligodendrocytes and astrocytes.

As reported above, the present inventors have also discovered that β-amyloid peptide and other reagents that induce the formation of ROS maybe used to test for the presence of precursor in a body fluid or tissue sample. Thus, the diagnostic methods arc amenable to the use of not only FeSO₄, but other reducing agents and/or oxidizing agents as well, particularly other classical Fenton reactants also exemplified by FeCl₃ and H₂O₂.

Although there are several conceivable methods for accurately measuring quantities of DHEA that are formed by the method of the invention, DHEA quantities are preferably measured by (i) extracting the samples with an organic solvent, (ii) subjecting the extracted material to HPLC chromatography, and (iii) detecting the DHEA using radioimmunoassay. A preferred organic solvent is ether:ethylacetate (v/v), but any solvent may be used and many are known in the art. Radioimmunoassay kits for measuring DHEA and its sulfated derivative, DHEA-S, are commercially available (see for example Diagnostic Systems Laboratories, 445 Medical Centre Blvd., Webster, Texas 77598). Antibodies specific for DHEA are also available from ICN Pharmaceuticals (Costa Mesa, CA). Alternatively, quantities of DHEA may be measured by gas chromatography coupled to mass spectrometry. Other studies have optically measured steroid levels in initial extracts, without any further purification steps. The present inventors have found, however, that attempting to measure DHEA and other neurosteroids without previous purification procedures may not provide accurate measurements.

Given the known characteristics of the alternative metabolic pathway for DHEA, the methods of the invention may be supplemented with further assays to verify the validity of the results. For instance, the samples may also be tested for an increase in radical oxygen species after treatment with the regulating agent, e.g., by using 2,7 DCF fluorescence. The correlation between the treatment and the effect may also be verified by determining whether the effect is blocked by vitamin E. The samples may also be tested directly for the presence of the metabolic precursor for DHEA once it has been identified.

The diagnostic method described above may also include steps whereby the cells are treated with compounds that optimize the synthesis of DHEA using the alternative pathway. For instance, SU 10603 is a known inhibitor of P450c17 (64). P450c17 is the enzyme responsible for the classical conversion of pregnenolone to DHEA (the non-oxidative stress-induced pathway). Thus, if a test sample of cells employed for the present methods has the ability to synthesis DHEA via both pathways, treating with SU 10603 would clarify results by inhibiting synthesis via the classical pathway such that only the potential for the alternative pathway is measured.

Trilostane (Stegram Pharmaceuticals, Sussex, U.K.), a specific inhibitor of 3β-HSD, also inhibits the metabolism of pregnenolone by inhibiting its conversion into progesterone. By treating a cell sample with both trilostane and SU 10603, it is possible to maximize pregnenolone production. By treating with mevastatin, a 3-hydroxy-3-methylglutaryl CoA reductase inhibitor, i.e., an inhibitor of cholesterol synthesis, it may be possible to modulate the levels of the unknown substrate.

As described above, the present inventors have surprisingly discovered that DHEA levels are significantly higher in the brains of Alzheimer's patients than in the brains of control patients, and that concomitant to this increase is a decrease in the precursor for the oxidative stress-induced metabolic pathway for DHEA. Oxidative stress contributes to a variety of neurological disorders, and it is likely that elevated DHEA levels may be used as an indicator for a variety of such neuropathologies, including dementias in general, Alzheimer's disease, Parkinson's disease, ALS (Amylotropic Lateral Sclerosis), amyloidosis, Ataxia Telangiectasia, Binswanger's disease, brain cancer (which, in some cases, may lead to increased reactive oxygen species), Hallervoden-Spatz disease, Huntington's disease, Krabbe disease, Leigh's disease, mitochondrial disorders, Multi-Infract dementia, Pelizaeus-Merzbacher disease, Pick's disease, stroke, and traumatic brain injury. In fact, diagnosing traumatic brain injury may particularly benefit from the disclosed diagnostic assays due to the dramatic acute increase of oxidative stress due to the injury. Spinal cord injury and other peripheral nervous system injuries may also be diagnostic targets given the ability of the spinal cord Swann cells to make neurosteroids and the increased oxidative stress due to the injury,

Thus, the present invention generally includes a method of diagnosing the existence of a neuropathologic disorder in a patient comprising measuring the quantity of a neurosteroid in the CNS of said patient. Depending on whether one measures DHEA levels or levels of the precursor, either an increased quantity or a decreased quantity of neurosteroid relative to a control will indicate the development of a neuropathologic disorder.

It has long been speculated that DHEA may be important in the aging process, particularly in modulating memory formation via its actions on NMDA receptors in the hippocampus. - We have demonstrated that, contrary to current theories, levels of DHEA are much higher in the Alzheimer's disease brain than in the normal brain. The question remains, however, as to whether DHEA synthesis plays a protective role in neuropathology, or is acting to potentiate the disease process.

For instance, it has been shown that DHEA and its sulfated derivative are neuroprotective against excitaory amino acid-induced toxicity in the hippocampus (32).

However, little research has been done on the neuroprotective effects of DHEA on glia. It could be that the observed increase in DHEA formation by glia in response to oxidative stress may serve a protective role. Alternatively, given the observation that β-amyloid peptide has the potential to stimulate the oxidative strees-induced synthesis of DHEA, it may be that astrocytes become reactive to this peptide in Alzheimer's disease pathology and produce extracellular matrix molecules and growth factors that help to potentiate plaque formation (33). In this scenario, the increase in DHEA would be a harmful event, helping to protect reactive astrocytes and potentiate neuronal damage. It remains to be seen whether DHEA plays an important role in neuropathology, or whether it is simply an epiphenomenon of the disease process.

Regardless of whether DHEA plays an important role in the pathogenesis of Alzheimer's disease and other neuropathologies, it can serve as a useful marker of disease via the diagnostic methods described above. But if DHEA is found to lay a protective role, the novel observation that the alternative metabolic pathway may be stimulated by exposure of cells to β-amyloid peptide may provide a new means of synthesizing DHEA via cells that are capable of the alternative metabolic pathway, for use in pharmaceutical compositions. U.S. Patent No. 4,812,447, herein incorporated by reference, provides a discussion of the use of DHEA and DHEA-S in pharmaceutical compositions for the treatment of Alzheimer's diease.

Thus, the present invention also includes a method of increasing or causing DHEA synthesis by cells in vitro comprising exposing said cells to β-amyloid peptide, wherein such exposure results in synthesis of DHEA. Because brain cells are the only cells at present demonstrated to facilitate this mode of synthesis, the preferred cells for the synthesis are brain cells, and particularly astrocytes and oligodendrocytes. However, it is possible that other cells may be identified that may be used for such a method. Moreover, once the precursors and enzymes involved in the alternative metabolic pathway have been identified, it may be possible to create a cell-free system for synthesizing DHEA based on this finding,

The following methods and working examples serve to illustrate the concepts underlying the present invention.

### Experimental Procedures

### Cells lines

The human glioma cell line KG-1-C was obtained from the Riken Cell Bank (Tsukuba, Japan). The MGM-1 and MGM-3 cell lines were a gift from Drs. Hiroaki Kataoka and Mashi Koono (Miyazaki Medical College, Japan). Normal human astrocytes (NHA) were purchased from Clonetics (San Diego, CA), while purified human neurons (hNT-PF) were purchased from Stratagene (La Jolla, CA). The P450c17 antibody was a generous gift from Dr. Dale B. Hales at the University of Illinois. Trilostane, a specific inhibitor of 3β-HSD, was a gift from Stegram Pharmaceuticals, Sussex, UK. SU 10603, an inhibitor of P450c17 (64), was a gift from CIBA-Geigy, Basel, Switzerland.

### Tissue Samples

All human tissue samples and CSF were obtained from the Harvard Brain Tissue Resource Center in Belmont, MA. Samples for steroid measurements were either snap frozen or passively frozen in liquid nitrogen. Samples for RNA extraction were snap frozen. Mean age for all patients was 74.6 years for Alheimer's disease (AD) patients and 73.4 years for controls. Mean postmortem interval was 10.2 hours for AD patients and 14.7 hours for controls. Serum samples were obtained from Dr. J. R. Rapin (CEB, Mont Saint-Agnes, France). Protocols for the use of human tissue were approved by the Georgetown University Internal Review Board.

### Cell Culture

KG-1-C is derived from a mixed glioma and has been described as having the characteristics of an oligodendrocytoma (69). MGM-1 and MGM-3 are derived from glioblastoma multiforme tumors (70). All three cell lines were grown in Dulbecco's Modification of Eagle's Media (DMEM) with 10% fetal bovine serum (Mediatech Cellgro, Gaithersberg, MD) plus 100 U/ml Penicillin, 100 mg/ml Streptomycin and 1.25 mg/ml Amphotericin B (Sigma, St. Louis, MO) at 37C and 6% CO₂. NHA cells were grown at 37C and 6% CO₂ in Astrocyte Growth Medium (Clonetics, San Diego, CA) containing 5% fetal bovine serum, 0.02 mg/ml human epidermal growth factor, 25 mg/ml insulin, 0.025 mg/ml progesterone, 50.0 mg/ml transferrin, 50.0 mg/ml Gentamicin, and 0.05 mg/ml Amphotericin B. hNT-PF cells are a post-mitotic, differentiated cell derived from the Ntera2/D1 teratocarcinoma cell line (NT2). NT2 precursor cells can be induced to differentiate in vitro by six week treatment with retinoic acid (Stratagene, La Jolla, CA). hNT-PF cells were plated and maintained at 37C and 6% CO₂, in hNT-PF neuron conditioned media (Stratagene).

### Reverse Transcription-Polymerase Chain Reaction and DNA Blotting

Total RNA was isolated from each cell type by the acid-guanidium thiocyanate-phenol-chloroform extraction method using RNazolB (Tel-Test Inc., Friendswood, TX). RNA from each cell type was reverse transcribed using oligod(T)s and MuLV reverse transcriptase according to the protocol provided by Perkin Elmer (Foster City, CA). Primers specific for the genes of interest were used to amplify specific fragments (Table 1). PCR products were run on 1.5% agarose gels and transferred to nylon membranes (Amersham Pharmacia Biotech, Inc., Piscataway, NJ). Probes were synthesized by RT-PCR from either total human adrenal RNA (PBR, P450scc, P450c17 or 3b-HSD) or total human brain RNA (MBP). To confirm specificity, probes were sequenced using the ABI Prism protocol at the Lombardi Cancer Center Core Facility, Georgetown University Medical Center. Probes were labeled by random primed labeling using the kit from Boehringer Mannheim (Indianapolis, IN) and were incubated with the membranes overnight at 42°C. Membranes were washed and exposed to XOMAT-AR film (Eastman Kodak, Rochester, NY) for 2-24 hours at -70°C.

**Table 1. RT-PCR primer sequences and expected product size**

| Gene | Forward primer | Reverse primer | Product size (bp) |
|---|---|---|---|
| MBP | 5'-AATTCGCGTGTGGCAGAG-3' | 5'-ACGCCTTGTGCCTACTCGTTAC-3' | 420 |
| GFAP | 5'-ACCTTGGCACAGACACAATG-3' | 5'-GTGCTGGCTCGTCTTTATTC-3' | 586 |
| PBR | 5'-CTAACTCCTGCCAGGCAGTG-3' | 5'-TGACCAGCAGGAGATCCAC-3' | 401 |
| | | | |
| P450scc | 5'-CTGTTGGATGCAGTGTCTCG-3' | 5'-GTGCCATCTCATACAAGTGC-3' | 523 |
| P450c17 | 5'-CAACAACCGTAAGGGTATCG-3' | 5'-GGCTGAAACCCACATTCTG-3' | 701 |
| 3β-HSD | 5'-AGACATTCTGGATGAGCC-3' | 5'-AGAATTGACCTCGGACAC-3' | 578 |

### Immunocytochemistry

Cells were grown in four chamber slides (VWR, W. Chester, PA) and fixed with 70% ethanol at 4C for 15-30 minutes. Slides were incubated with primary antibody in 10% calf serum for one hour at room temperature at the following dilutions: PBR; 1:200, (34), P450scc; 1:200 (49), P450c17; 1:200, 3b-HSD; 1:100 (Oxygene Inc, Dallas, TX). HRP-linked anti-rabbit secondary antibody (Transduction Laboratories Inc., Lexington, KY) was applied at a dilution of 1:2000 for one hour at room temperature, and HRP substrate 3-amino-9-ethyl carbazole (Sigma, St. Louis, MO) was used to detect peroxidase reaction. Slides were counterstained with hematoxylin (Sigma, St. Louis, MO).

### Steroid Biosynthesis Experiments

*De Novo* Steroid Synthesis: All steroid synthesis experiments were performed with the cells at 60-80% confluency. In order to determine the ability of the cells to synthesize steroids *de novo,* cells were incubated for two hours at 37C and 6% CO₂ with specific steroidogenic enzyme inhibitors as previously described (50). In brief, endogenous cholesterol synthesis was blocked by incubating cells with 20 mM Mevastatin in serum-free media (Sigma, St. Louis, MO) for 1 hour. 10 mM Trilostane and 5 mM SU 10603 were then added. After 30 minutes, 3 ml ³H-mevalonactone (33 Ci/mmol, DuPont NEN, Boston, MA) and 10 mM cold mevalonactone (Sigma, St. Louis, MO) were added. Thirty minutes later, media and cells were combined and homogenized on ice for 20 sec. Homogenates were extracted twice with 4 ml of ether:ethyl acetate (1:1, v/v, Fluka, New York, and EM Science, Gibbstown, NJ) and evaporated. Extracts were reconstituted in 100% methanol and separated on a Beckman System Gold HPLC system, using a 126NM Solvent Module, 166NM Detector and a Beckman ultrasphere XL 3-mm Spherical 80 A pore column (Beckman, Fullerton, CA), eluted on a methanol gradient. Fractions were collected each minute and counted by liquid scintillation spectrometry (EG&G Wallac, Gaithersburg, MD) to determine production of radiolabeled steroids. Steroids were identified by respective retention times compared with radiolabeled standards (Retention time P = 28 min, Retention time progesterone = 24 min, Retention time D = 18 min). In previous experiments, steroids in these fractions were identified as P, progesterone and D respectively by Gas Chromatography-Mass Spectrometry (15).

Alternative Pathway: To determine the amount ofD synthesized by the alternative pathway cells were treated as previously described. (15). In brief, cells were washed and oligodendrocytes and astrocytes were incubated with increasing concentrations of FeSO₄ in serum free media, with and without SU 10603 (10 mM); neurons were incubated in serum free media containing 5 mM P plus FeSO₄ with and without 10 mM SU 10603. After two hours, cells and media were homogenized and extracted as described above. Extracts were run on C 18 TLC plates (Whatman, Clifton, NJ) with a mobile phase of 95% methanol (v/v); radiolabeled standards for P, progesterone, and D were run on each plate, and appropriate steroid fractions were collected for each sample. TLC fractions were extracted with 2 ml diethyl ether (Fluka, New York) and evaporated. TLC extracts were assayed by specific radioimmunoassay (RIA). In separate experiments, steroids eluted from TLC were further identified by HPLC, RIA and Gas Chromatography-Mass Spectrometry (data not shown).

Tissue Samples: For brain samples from Alzheimer's patients and control patients, samples were treated as previously described (17). In brief, aliquots of homogenates were incubated with a final concentration of 30 mM (brain) or 10 mM (serum and CSF) FeSO₄ at 37°C for 2 hours. After 2 hours, samples were extracted and purified as described for the endogenous levels, and assayed by specific radioimmunoassay.

Serum and CSF Levels of DHEA: Serum was obtained from 11 patients, 5 with AD and 6 age-matched controls. Serum samples were split and treated with and without 10 mM FeSO₄. Serum and CSF samples were extracted as described for the brain tissue samples, and purified by HPLC. DHEA levels were measured by specific radioimmunoassay.

β-Amyloid Regulation of the Alternative Pathway: MGM-1 cells were plated on 96 well (~5,000 cells/well, for ROS measurements) or 24 well (~15,000 cells/well, for D measurements) plates (Corning Costar, Acton, MA). At the same time, 1 mg Ab (Ab (1-42), AnaSpec Inc., San Jose, CA) was dissolved in 0.5 ml serum-free media and incubated at 4C for aggregation. 24 hours later, cells were incubated in DMEM with 10% fetal bovine serum and 50 mM Ab or 50 mM Ab and 10 mM Vitamin E (Sigma, St. Louis, MO). Control samples were incubated in DMEM with 10% FBS. Cells were incubated at 37C and 6% CO₂ for at least 12 hours and processed for ROS generation (see below) or D synthesis as described above for the alternative experiments.

### ROS measurements

Treatment media was removed and the cells were washed with PBS. The fluorescent probe 2,7- dichlorofluorescin diacetate (2,7-DCF, Molecular Probes, Inc., Eugene, OR) was used to measure levels of oxidative stress (48). Cells were incubated with 50 mM 2,7-DCF, fluorescence was measured at 485nm/535nm using the Victor² Multilabel Counter (EG&G Wallac, Gaithersburg, MD) and values of cellular fluorescence were obtained.

### Radioimmunoassay

RIA using antibodies specific for each steroid (ICN Pharmaceuticals, Costa Mesa, CA Mesa) was used to measure P, progesterone and D levels. The data were analyzed using the IBM-PC RIA Data Reduction Program (version 4.1) from Jaffe and Associates (Silver Spring, MD). Levels of β-amyloid species in AD and control brain samples were analyzed using 4G8 (Senetex PLC, Napa, CA), a monoclonal antibody to β-amyloid peptide that recognizes amino acids 17-24. Samples in 0.25 M sucrose buffer were run on 4-20% precast gradient gels (Novex, San Diego, CA). Samples were transferred to nitrocellulose membranes and incubated in primary antibody for 1 hour at room temperature at a dilution of 1:2000. Membranes were incubated in secondary antibody (1:1000) for 1 - 1.5 hours at room temperature and blots were visualized using ECL reagents (Amersham Pharmacia Biotech, Pisquataway, NJ). Blots were exposed to XOMAT film.

### Protein Assay

Microgram amounts of protein were measured using the dye-binding assay of Bradford (1976) (39), using bovine serum albumin (ICN Pharmaceuticals, Costa Mesa, CA) as the standard.

### Statistics

Statistical analysis of the alternative pathway results using cell lines was performed by one-way ANOVA using the INSTAT 3.00 package from GraphPad (San Diego, CA). Analysis of ROS and b-amyloid data was done using unpaired Student's t test on SigmaPlot (Jandel Scientific, San Rafael, CA). Statistical data from tissue samples is shown as means ± SEM from 3-15 measurements. Data were analyzed using Welch's t test, with normality tests. Significance level was set as p < 0.05.

### Results: Characterization of neurosteroid synthesis and regulation using brain cell lines

### Characterization of cell types

To determine the phenotypes of the glioma cell lines, we performed RT-PCR for MBP, an oligodendrocyte marker, and GFAP, an astrocyte marker. We found the all three glioma cell lines express message for MBP (data not shown), as does total human brain RNA. None of the glioma cell lines express message for GFAP (data not shown). These cells will be collectively referred to as oligodendrocytes.

NHA cells from Clonetics are shipped immunopositive for GFAP and we confirmed GFAP expression by RT-PCR (data not shown). These cells are also immunonegative for CD68, a microglial marker, and for 2',3'-cyclic nucleotide-3'-phosphodiesterase, an oligodendrocyte marker (Clonetics Certificate of Analysis, Clonetics, San Diego, CA). The hNT-PF cells are derived from a Ntera2/D1 teratocarcinoma cell line by 5-6 weeks of differentiation by retinoic acid. These cells are post-mitotic and have been well characterized by other investigators. Once differentiated, they express the three neurofilament proteins, but not GFAP (61). They also express adhesion molecules specific to neurons, such as NCAM and N-cadherin (77). Electrophysiologically, hNT-PF cells behave like neurons, with tetrodotoxin-sensitive sodium currents (78). Retinoic acid differentiation also induces the expression of functional glutamate receptors, of both the NMDA and non-NMDA subtypes (86).

### Oligodendrocytes synthesize P de novo, whereas astrocytes and neurons do not

A steroid-synthesizing cell is defined as a cell that is able to convert endogenous cholesterol to pregnenolone (P). Very few cells in the body have this ability - it is limited to steroidogenic tissues such as the adrenal glands, gonads, placenta and brain. However, almost all cells have the ability to metabolize steroids to final steroid products. We blocked endogenous cholesterol synthesis with the 3-hydroxy-3-methylglutaryl-CoA reductase inhibitor Mevastatin and incubated the cells with a radiolabeled cholesterol precursor, ³H-mevalonactone. By blocking the metabolism ofP by Trilostane and SU 10603, we attempted to maximize P production. HPLC separation of the samples indicated that the MGM-3 cell line synthesizes P *de novo* from a radiolabeled precursor. In some experiments, KG-1-C cells also made radiolabeled P, but when results from multiple experiments were combined, this effect was not significant. In addition, KG-1-C and MGM-3 cells made radiolabeled D, indicating that SU 10603 was not effective in blocking endogenous D production in these two cell lines. This suggests that the mechanisms involved in human brain steroid biosynthesis may be different than those present in adrenal and gonads. MGM-1, NHA and hNT-PF cells do not make P *de novo* (data not shown).

We hypothesized that high endogenous levels of ROS might be responsible for the *de novo* D formation in KG-1-C and MGM-3 cells. Human glioma cells were treated for at least twelve hours with or without 10 mM Vitamin E in DMEM with 10% fetal bovine serum. We measured ROS and D levels in these cells as described in the Experimental Procedures. We found that Vitamin E treatment of these cells lowered both ROS and D levels as compared to untreated cells (data not shown). ROS and D levels across all treatments were correlated, with r = 0.7034.

### Human brain cells express mRNA and immunoreactivity for different components of the peripheral steroid biosynthesis machinery

In order to determine possible mechanisms of steroid biosynthesis in human brain cells, we looked at the expression of four components of the classical peripheral steroidogenic pathway. Cells were assayed for the presence of mRNA for PBR, P450scc, P450c17 and 3β-HSD. All three glioma cell lines express mRNA for all four components (data not shown). In addition, the corresponding proteins were detected by immunocytochemistry in these cells (data not shown). KG-1-C and MGM-1 cells express immunoreactivity for all four components. Interestingly, MGM-1 cells show a distinct nuclear localization for PBR. This corresponds to recent data from our laboratory showing that in more aggressive human breast cancer cell lines and biopsies PBR was found to be localized in the nuclear and perinuclear area in contrast to the mitochondrial localization seen in non-aggressive cells (53). MGM-3 cells express immunoreactivity for PBR, P450scc and P450c17, but not for 3β-HSD. However, MGM-3 does have message for 3β-HSD. This may indicate an increased breakdown in 3β-HSD mRNA or increased degradation of the protein. As a whole, these data indicate that in human oligodendrocytes, mRNA and protein expression corresponds with the ability of the cells to synthesize neurosteroids *de novo.*

Human astrocytes express mRNA for all four components (data not shown). They are immunoreactive for PBR, P450scc and P450c17, although protein expression is much lower then that seen in the glioma cells. Similar to MGM-3 cells, NHA have mRNA but no immunoreactivity for 3β-HSD. Again, this may indicate alterations in mRNA or protein stability. NHA cells have the potential to synthesize P and D, but not progesterone. Human neurons express mRNA for PBR, P450c17 and 3β-HSD, but not for P450scc, while the NT2 precursor cells have message for all four components (data not shown). This indicates that hNT-PF will not be able to synthesize P *de novo,* in agreement with the data above, but they may be able to metabolize P to other neuroactive steroids.

### Oligodendrocytes and astrocytes possess an alternative pathway for D synthesis; neurons do not have the alternative pathway.

We recently demonstrated that rat glioma cells are able to synthesize neurosteroids, such as DHEA (D), through a P450c17-independent pathway (15). We used a similar protocol to assess alternative pathway activity in human brain cell lines. We found that the MGM-1 cell line has the ability to make D via the alternative pathway (Fig. 6B, see brief description of drawings for one-way ANOVA F and p values), although these cells can not make P *de novo* from a radiolabeled precursor. FeSO₄ did not significantly affect the KG-1-C and MGM-3 D levels (Fig. 6A, 6C). It should be noted that KG-1-C and MGM-3 are the cells that synthesized radiolabeled D *de novo* in a manner which was not inhibited by SU-10603, again suggesting that endogenous free radicals may be responsible for D formation in these cells. FeSO₄ treatment had a significant effect on D formation in NHA cells (Fig. 6D), indicating that this effect is not limited to tumor cell lines. This effect was only seen at the highest dose of FeSO₄ used, indicating that there may be differences between cell types in terms of their sensitivity to FeSO₄ or ROS levels.

The hNT-PF cells were also incubated with increasing concentrations of FeSO₄, with and without SU 10603 as described above. In addition, these cells were incubated with 5mM P to enhance D production; recent studies have indicated that providing P as a substrate can enhance the effects of FeSO₄ in rat C6 cells (15). Human neurons were not affected by FeSO₄ treatment (Fig. 6E), suggesting that this alternative pathway is specific to glial cells. hNT-PF cells were also not affected by the SU 10603 treatment, indicating that D in these cells does not come from endogenous activity of the P450c17 enzyme. Neuronal D, therefore, may be completely derived from other sources.

We should note that in all human brain cells examined SU 10603 did not inhibit D formation. SU 10603 has a very complex pharmacology; in some instances, it seems to increase D levels (Fig. 6D). However, this is the only available inhibitor of P450c17 activity known. In contrast to the results presented here, this compound inhibited P450c17 activity in testicular Leydig cells (data not shown) indicating that the drug can suppress P450c17 activity.

### Treatment of cell lines from human brains with FeSO₄ causes an increase in intracellular free radicals

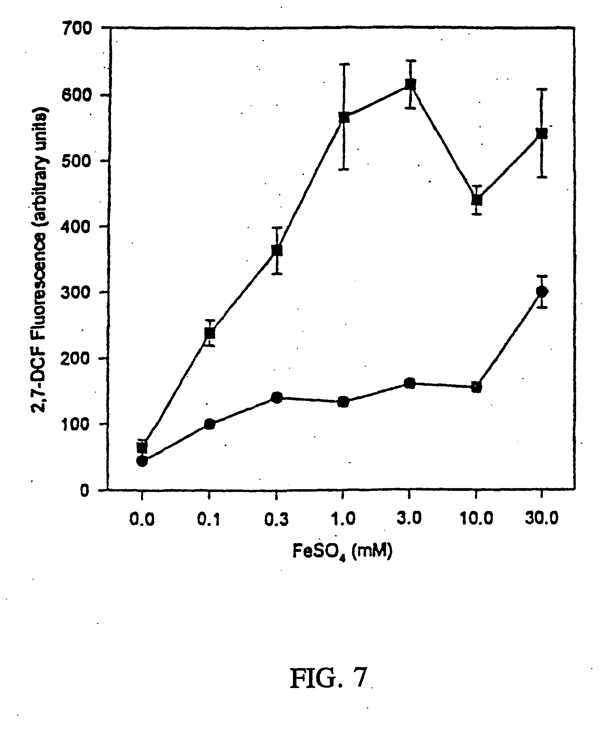

We hypothesized that a potential mechanism by which FeSO₄ regulates D synthesis is by the regulation of cellular ROS production. We treated MGM-1 human glioma cells and NHA cells with FeSO₄, and measured ROS levels in these cells according to the methods described in the Experimental Procedures. We found that all concentrations of FeSO₄ tested caused a significant increase in ROS levels above untreated cells (Fig. 7, see brief description of drawings for one way ANOVA F and p values). NHA cells responded more robustly to FeSO₄ then did MGM-1 cells. This indicates the FeSO₄ does cause increases in ROS in these cells, and makes this mechanism a potential regulator ofD synthesis.

### Treatment with β-Amyloid increases ROS and D levels in MGM-1 human glioma cells

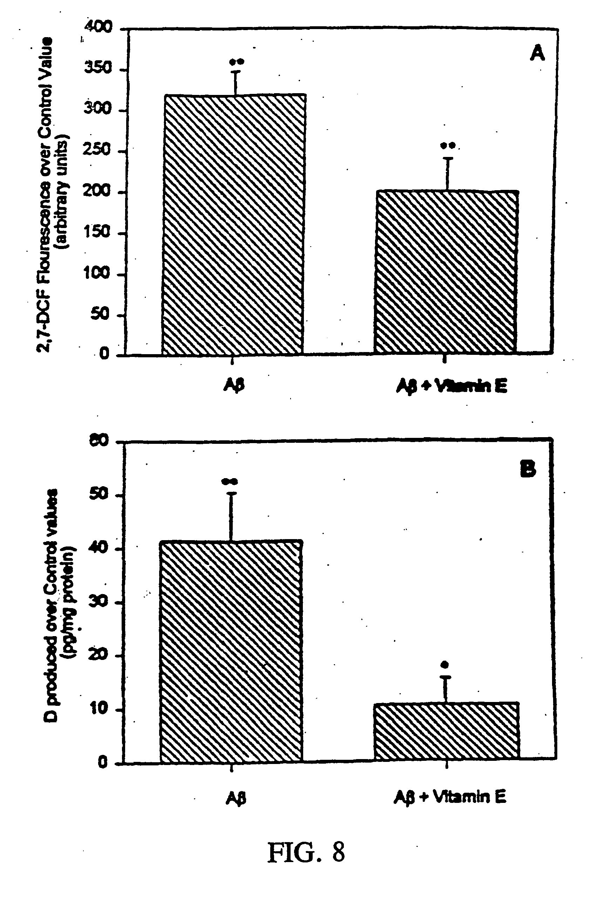

We showed that endogenous cellular ROS may be responsible for the *de novo* D formation seen in KG-1-C and MGM-3 cells, and that addition of Fe²⁺, which induce ROS formation (Fig. 7), caused D formation in MGM-1 and NHA cells. In order to determine if increases in ROS caused by other treatments could enhance D formation, we treated MGM-1 cells with 50 mM Aβ, with and without 10 mM of the antioxidant Vitamin E. We found that treatment with Ab caused a significant increase in 2,7-DCF fluorescence (p < 0.01, Student's unpaired *t* test) versus control levels, indicating an increase in ROS (Fig. 8A). This increase was partially blocked by concurrent treatment with Vitamin E (p = 0.054), but was still significantly above control levels (p < 0.01). Treatment with Ab also increased D levels significantly over control (p < 0.01, Fig. 8B). This significant increase was blocked by treatment with Vitamin E (p < 0.05 versus Ab). This data suggests that ROS may play a role in regulating D synthesis in human brain.

### Discussion

Although there is a considerable body of evidence indicating that in rodents, steroids found in the brain may be of both peripheral and local origin, nothing is known about the ability of human brain to synthesize neurosteroids. Thus, reported herein is the first demonstration of steroid biosynthetic mechanisms in human brain cells. We examined the *de novo* biosynthesis of P from a radiolabeled precursor, mRNA and protein expression of PBR, P450scc, P450c17 and 3b-HSD, and presence of an alternative, free radical-mediated, pathway for D synthesis.

We demonstrate *de novo* synthesis ofP in oligodendrocytes, but not in astrocytes or neurons. This confirms data generated in the rat model, which indicate that oligodendrocytes are the probable source of brain-synthesized P (62), and validates the use of the rat as a model to study neurosteroid biosynthesis. However, we have not ruled out the possibility that astrocytes or neurons have the ability to metabolize P into progesterone and D, or other neuroactive compounds. Oligodendrocytes can be designated as steroid synthesizing cells, and astrocytes and neurons as steroid metabolizing cell populations. This means that neuroactive steroids, which may play a role in neuronal excitability, can be produced locally from a number of different cellular sources, as well as coming from the periphery.

We examined mRNA presence and immunoreactivity for four components of the steroid biosynthesis machinery. Oligodendrocytes have mRNA and immunoreactivity for PBR, P450scc, P450c17 and 3b-HSD. This confirms the ability of oligodendrocytes to make cholesterol from a precursor, transport cholesterol into the mitochondria and metabolize it to P. Enzymatic activity for P450c17 was not detected, due to the inability of the specific inhibitor SU 10603 to block D formation in these cells (Fig. 6A-C). Astrocytes also have mRNA and immunoreactivity for PBR, P450scc and P450c17, although we were unable to find activity for P450scc. There may be co-factors necessary to P450scc function not present in astrocytes, thereby preventing enzyme activity. These results indicate a possible role for astrocytes as steroid-metabolizing cells that may make neuroactive steroids from the oligodendrocyte-derived P. Astrocytes can potentially convert P to D by the P450c17, although the P450c17 inhibitor SU 10603 (64) has no effect on D levels in these cells (Fig. 6D). In both the oligodendrocytes and the astrocytes, immunoreactive P450c17 did not have any discernable enzymatic activity. Similarly to P450scc in the astrocytes, there may be co-factors required for this enzyme function that are not available in these cell types. Interestingly, in all glial cells, there seem to be two isoforms of P450c17 identified by the DNA blot. A previous study has also found two transcripts of P450c17 in rat cortex and cerebellum (81). There may be preferential translation of one isoform or the other, and these isoforms may have different activities. Finally, neurons express mRNA for PBR, P450c17 and 3b-HSD, but not for P450scc. This would indicate that these cells are unable to make P *de novo,* as supported by our *de novo* experiments. It remains to be seen if neurons metabolize P to other steroids.

There is controversy in the field on the mechanism of D biosynthesis in the brain. In 1994, Lieberman and colleagues proposed the existence of alternative precursors of the neurosteroids present in rat brain extracts that could react with compounds unrelated to peripheral steroid biosynthesis, and produce neurosteroids. They demonstrated that treatment of organic extracts of rat brain with different oxidizing compounds resulted in the liberation ofD (28). We have demonstrated that rat glioma cells, devoid of P450c 17 mRNA and protein, when treated with different oxidizing compounds also formed D (15). This alternative pathway was found to be specific to cells from the brain, and could not be replicated in a cell-free environment (15). Now we report the presence of this alternative pathway in cell lines from human brain.

Oligodendrocytes and astrocytes can make D by an alternative pathway. There also seems to be a threshold for ROS induction of D synthesis, which may vary between oligodendrocytes and astrocytes. Astrocytes seem to produce more ROS in response to Fe²⁺ than glioma cells, which may indicate an increased sensitivity to FeSO₄ and potentially, a higher threshold for ROS regulation of D synthesis in normal human brain cells. These data suggest that the oxidative environment of the brain under different conditions may influence D formation by glia. Neurons do not have the alternative pathway for D synthesis suggesting that the alternative pathway is specific to glia. Oligodendrocytes that do not respond to Fe²⁺ can synthesize D *de novo* in a P450c17-independent manner. This is potentially due to high endogenous ROS. Interestingly, Vitamin E treatment of the glioma cell lines decreases MGM-3 ROS and D levels to those seen in MGM-1 cells, but KG-1-C cells are less drastically affected by this antioxidant. These data also confirm results in our laboratory in which we found that isolated rat oligodendrocytes and astrocytes in a primary culture system produced D in response to Fe²⁺ (42).

It should be noted that it is customary to study enzyme activities in steroidogenesis models by adding the ideal precursor, i.e. P, for D formation via P450c17. Because we believe that by adding P we may bias the process towards a P450c17-mediated D formation, which may not be the physiological mechanism, we opted to perform the studies reported above in the absence of the precursor. We used P only in hNT-PF cells, where because of the limited number of cells, we chose to use optimal conditions. Under these conditions P was not metabolized to D by the P450c17 enzyme. It should be noted that addition of 5 µM P to oligodendrocytes or astrocytes did not result in increased D production (data not shown).

We use SU 10603 as the means to inhibit the endogenous unbiased P450c17 enzyme activity. Although this compound was developed as a specific inhibitor for P450c17, it has been shown to inhibit other P450s, including P450scc (60). We have found that addition of SU 10603 alone has no effect on P levels in any of the cell lines used (data not shown), indicating that the drug is not altering P450scc activity. Considering that SU 10603 did not inhibit D formation in any human brain cell system tested we can conclude that P450c17 is not the mechanism of D formation in human brain cells.

As noted above, the presence of P450c17 activity in rodent brain has been a controversial issue. In recent studies, we found expression of P450c17 mRNA and protein in rat oligodendrocytes but not in astrocytes where the alternative pathway is responsible for D formation (42 and unpublished data). However, Zwain and Yen recently reported that D could be formed in rat brain, and more specifically by astrocytes and neurons, via the P450c17 pathway (87). Because of the diverse technology used to isolate and characterize D, i.e., HPLC, TLC and GC-MS followed by RIA (15, 42, and the present report) versus direct RIA (87) it is difficult to compare at present the data obtained in the rat and the data reported herein.

In order to determine if ROS generation was involved in regulating the alternative pathway in a physiological context, we treated MGM-1 cells with Aβ, a component of Alzheimer's disease plaques. Aβ is toxic to neurons and causes an increase in cellular ROS (54). This effect can be blocked by antioxidants such as Vitamin E (52). We found that Aβ treatment increased cellular ROS in MGM-1 human glioma cells, and increased D levels. Both the increases in ROS and D could be blocked by co-treatment with Vitamin E. This is the first demonstration of a regulatory mechanism for D synthesis in the brain, and will enable us to gain insight as to the role of D in human brain, particularly in neuropathology.

It is known that Aβ, while toxic to neurons, induces astrocytic activation (56) and cytoskeletal reorganization (80), but does not alter astrocyte viability (76). It has been hypothesized that astrocyte activation and subsequent deposition of chondroitin sulfate proteoglycans (33) and growth factors (90) may be involved in plaque formation. It is possible that glial cells may be able to resist the toxic effects of Ab by their ability to produce D, known to exert neuroprotective effects in the hippocampus (32).

It is known that PBR is upregulated in human brain tumors (43, 68), which may indicate a general upregulation of the steroid biosynthetic pathway. Furthermore, PBR binding sites are significantly increased in specific areas from AD brains, as well as brains from patients with Parkinson's disease (66). Upregulation of steroid biosynthetic components may be, like the activity of the alternative pathway, a sign of altered homeostasis, neurodegeneration or trauma in the brain. It may be that an increase in neurosteroid biosynthesis is a hallmark of an altered or toxic cellular environment. The alternative pathway may only become a player in brain steroid biosynthesis in situations in which normal homeostasis has been disrupted, as in the MGM-1 cells which have nuclear PBR and therefore cannot synthesize neurosteroids via the classical enzymatic pathway. Thus, alterations in cellular processes due to injury or disease may allow for the function of the alternative pathway in cells that do not normally use this pathway.

### Example 1

We measured levels of pregnenolone (P) and DHEA (D) in samples from human hypothalamus, hippocampus and cortex from AD patients and age-matched controls. We found no significant difference in P levels in the hippocampus or frontal cortex (Figure 1). There may be a change in P levels in the hypothalamus, but due to the very small amount of P present in the samples, we were only able to make one measurement. There is a significant increase in D levels in all three areas (p < 0.01 vs. control levels, Figure 1). Because β-amyloid (Aβ) can also induce D formation in human glioma cells, presumably by increasing levels of intracellular ROS, this increase in D in AD patients is probably due to an increased level of oxidative stress in the AD brain.

We examined the expression of P450c17, the enzyme responsible for D formation in the periphery, in the human cortex and hippocampus using RT-PCR followed by southern blot with specific P450cl7 radiolabeled probes. We found that, although there is expression of P450c17 in the-cortex in both AD (in one out of two patients) and control patients, there is no expression in the hippocampus (Figure 2). This strongly suggests that any D found in the hippocampus comes from alternative pathway activity, not endogenous P45Oc17 activity.

We also looked at the levels of Aβ in human brain samples by immunoblot analysis. Using the monoclonal antibody 4G8 we found that there is Aβ-immunoreactivity in the hypothalamus of both AD. and control patients (Figure 3A). However, Aβ-immunoreactivity present in control patients is a higher molecular weight species, at about 70 kDa, than that in the AD hypothalamus, at about 50 kDa. Recent studies have demonstrated that the form of Aβ that is toxic to neurons is of a lower molecular weight. Higher molecular weight complexes are not toxic (27).

In the hippocampus, AD samples have a much higher Aβ load (Figure 3B), with molecular weights ranging from 45-80 kDa, while control samples have very little detectable Aβ-immunoreactivity. This supports the hypothesis that D in the hippocampus is formed as a result of oxidative stress, induced by the higher levels of Aβ present. In the cortex, AD patients have a relatively constant level of Aβ immunoreactivity, ranging from 20-50 kDa. Control patients also have detectable Aβ-immunoreactivity, but these patients tend to have higher molecular weight species, ranging from about 30-50 kDa.

We next examined the ability of AD and control brain to make P and D via the alternative pathway, after treatment with 30 mM FeSO₄. We found that FeSO₄ does not significantly affect P levels in any area in control patients, although there is the suggestion of a strong affect in the hypothalamus. Treatment significantly increased P levels in AD hypothalamus and frontal cortex, but not in AD hippocampus (Table 2). D levels were more responsive to Fe²⁺ treatment. 30 mM FeSO₄ caused a significant increase in D in the hippocampus and cortex of control patients, but had no effect in the hypothalamus. AD patients showed a significant increase in D in the cortex, but no significant change in the hippocampus or hypothalamus.

We measured D levels in CSF of control and AD patients to determine if the increase in AD brain is reflected in the CSF. We found a 35-fold increase in D levels in AD CSF versus control CSF (Figure 4A). This is an enhancement of the 2-4 fold increase seen in the brain (Table 2).

**Table 2. Pregnenolone and DHEA levels in brain samples from AD and Control Patients with and without Treatment with FeSO₄**

| Sample area | P (pg/mg protein) | | D (pg/mg protein) | |
|---|---|---|---|---|
| | Untreated | 30 mM FeSO4 | Untreated | 30 mM FeSO4 |
| hypothalamus | C 199.9^{a} | 7707.2 ± 717.1 | 48.8 ± 8.6 | 61.4 ± 14.2 |
| | AD 1135.5 ± 200.6 | 6640.3 ± 245.6 | 118.9 ± 10.0^{##} | 180.8 ± 23.9^{##} |
| hippocampus | C2155.1 ± 890.4 | 8529.3 ± 2236.5^{NS} | 73.8 ± 10.9 | 128.3 ± 17.0^{*} |
| | AD 6384.7 ± 2922.0 | 11584.0 ± 2328.7 | 321.2 ± 77.9^{##} | 241.7 ± 48.2^{#} |
| frontal cortex | C 2466.5 ± 668.9 | 7339.8 ± 2150.4^{NS} | 36.4 ± 7.0 | 70.0 ± 11.2* |
| | AD4245.4 ± 1705.1 | 10485.5 ± 904.8^{**} | 131.4 ± 19.0^{##} | 219.3 ± 23.3^{**##} |

| | | | | |
|---|---|---|---|---|
| * p<0.05 vs. untreated patient ** p<0.01 vs. untreated patient # p<0.05 vs. control patient ## p<0.01 vs. control patient ^{NS} Non-significant vs. untreated patients ^{a} Due to the small amount of steroid present in this sample, we were only only to make one measurement | | | | |

Furthermore, Aβ-immunoreactivity, as detected by immunoblots, is increased in CSF from patients diagnosed with AD as compared to control patients (Figure 4B). We examined serum levels of D and the effects of 10 mM FeSO4 on D in the serum. Although there is a general decrease in serum D levels in AD as compared to control patients, this decrease is not significant in the present study. FeSO₄ caused a 1.5-3 fold increase in D levels in control serum, but had little effect on AD serum (Figure 5). There is a wide patient-to-patient variability in serum D levels, but the increase in D in control patients in response to FeSO₄ occurs in all samples.

### Discussion

We measured levels of DHEA (D) and pregnenolone (P) in Alzheimer's disease (AD) and control brain to determine if there are any significant changes in neurosteroid profiles with the development of AD. We found that D is significantly increased in AD brain in all three areas examined, while P levels are not significantly affected. This is another reflection of increased oxidative stress in the AD brain, potentially due to the actions of β-amyloid (Aβ). Other studies have shown increased carbonyls in neuronal cytoplasm and in nuclei of neurons and glia from AD brain (29), as well as increases in lipid peroxidation, protein peroxidation and disruption of mitochondria energy metabolism in AD (17, 19-21), indicating a role for reactive oxygen species in the development of AD.

We looked at expression of P450c17 in human brain to analyze the origin of brain D. We found that, while there is message for P450c17 in the frontal cortex, there is no detectable expression of P450c17 in the hippocampus. This indicates that D in the hippocampus is either derived from peripheral sources and accumulated and stored in the hippocampus, or derived from activity of the alternative pathway. Although we did not measure the peripheral serum D levels from these patients, published reports have indicated levels of peripheral D from 0.3-2 µg/ml (2), 1.83 nmol/l (30, age = 76-93) in healthy aged people (age 60-90) and ~0.2-60 nmol/l in patients aged 3-85 years not taking steroids (26). Our measured levels of D are higher then those reported for the serum, both in control and AD subjects, and are comparable to (P) or lower than (D) those levels previously reported (31). The higher level of D in AD hypothalamus, hippocampus and frontal cortex as compared to controls indicates that D can be derived by alternative pathway activity triggered by increased oxidative stress. Furthermore, there are increased levels of Aβ-immunoreactive species in the AD brains, particularly of lower molecular weight species, which have been shown to be toxic (27).

We examined the potential for alternative pathway activity in AD and control brain. We found that treatment with FeSO₄ causes a significant increase in P in control hypothalamus and in AD hypothalamus and frontal cortex. FeSO₄ causes a significant increase in D in the hippocampus and cortex of control patients, indicating the presence of an alternative precursor in these areas (28). There is no effect in the hypothalamus. AD patients show a significant increase in D only in the frontal cortex, indicating that the precursor of the alternative pathway is present there, but not in the hypothalamus or hippocampus. The higher levels of D present before FeSO₄ treatment suggest that, in AD brain, the precursor of the alternative pathway has already been converted. This results in a higher endogenous level of D in the AD brain, and a smaller effect of FeSO₄.

The increased levels of D in the AD brain are contrary to the current thinking on the role of D in aging. The decrease in D levels with age has been considered to be a hallmark of the process, potentially playing a role in a number of the problems occurring with aging. D replacement has been the focus of a number of clinical studies attempting to prevent age-related cognitive changes and dementia (23-26). We now show that the actual CNS levels of D are increased in AD pathology. This is the opposite of what has been seen in the periphery with normal aging. This observation has been used as the basis of the present invention to formulate diagnostic methods and kits to test for the early development of AD.

One of the major problems with AD diagnosis and treatment is the inability of clinicians to determine the onset of the disease. Currently, this is done by a combination of MRI scans, to look for generalized shrinkage of the brain and cognitive tests to determine state of dementia. Typically symptoms do not occur until very late in the disease process. If the changes in D in the CSF are indeed regulated by oxidative conditions within the brain, there may be alterations in CSF D early in the progression of the disease. We have also shown that presence of the alternative precursor can be detected in the serum of control patients, but not of AD patients. Further analysis of the data shown in Table 2 indicates that the serum shows a greater response to FeSO₄ treatment than does any other brain area tested. Therefore, by determining whether or not the alternative precursor is present in the blood or CSF indirectly by FeSO₄ treatment, one should be able to predict the conditions of oxidative stress in the brain. This indicates that the availability of the alternate precursor for D could be used as a diagnostic tool to determine the onset and progression of AD.

### REFERENCES

1. Parker, C.R. Jr (1999) Dehydroepiandrosterone and dehydroepiandrosterone sulfate production in the human adrenal during development and aging. Steroids 64: 640-647.
2. Baulieu, E.-E. (1996) Dehydroepiandrosterone (DHEA): a fountain of youth? J Clin. Endo. Metab. 81: 3147-3151.
3. Kroboth, P.D. et al. (1999) DHEA and DHEA-S: a review. J Clin. Pharmacol 39: 327-348.
4. Corpechot, C., P. Robel, M. Axelson, J. Sjovall and E.-E. Baulieu (1981) Characterization and measurement of dehydroepiandrosterone sulfate in rat brain. Proc. Natl. Acad. Sci. USA 78: 4704-4707.
5. Akwa Y. et al. (1991) Neurosteorids: biosynthesis, metabolism and function of pregnenolone and dehydroepiandrosterone in the brain. J. Steroid Biochem. Metab. 40: 71-81.
6. Majewska, M.D., S. Demirgoren, C.E. Spivak and E.D. London (1990) The neurosteroid dehydroepiandrosterone sulfate is an allosteric antagonist of the GABAA receptor. Brain Res. 526: 143-146.
7. Bergeron, R., C. de Montigny and G. Debonnel (1996) Potentiation of neuronal NMDA response induced by dehydroepiandrosterone and its suppression by progesterone: effects mediated via sigma receptors. J. Neurosci. 16: 1193-1202.
8. Baulieu, E.-E. and P. Robel (1996) Dehydroepiandrosterone and dehydroepiandrosterone sulfate as neuroactive neurosteorids. J. Endocrinology 150: S221-S23.
9. Flood, J.F., J.E. Morley and E. Roberts (1992) Memory-enhancing effects in male mice of pregnenolone and steroids metabolically derived from it. Proc. Natl. Acad. Sci. USA 89: 1567-1571.
10. Yoo, A., J. Harris and B. Dubrovsky (1996) Dose-response study of dehydroepiandrosterone sulfate on dentate gyrus long term potentiation. Exp. Neurology 137: 151.
11. Compagnone, N.A., A. Bulfone, J.L.R. Rubenstein and S.H. Mellon (1995) Steroidogenic enzyme P450c17 is expressed in the embryonic CNS. Endocrinology 136: 5212-5223.
12. Mellon, S.H. and C.F. Deschepper (1993) Neurosteroid biosynthesis: genes for adrenal steroidogenic enzymes are expressed in the brain. Brain Res. 629: 283-292.
13. Stromstedt, M. and M.R. Waterman (1995) Messenger RNAs encoding steroidogenic enzymes are expressed in rodent brain. Mol. Brain Res. 34: 75-88.
14. LeGoascogne, C. et al. (1991) Immunoreactive cytochrome P45017α in rat and guinea pig gonads, adrenal glands and brain. J. Reprod. Fertil. 93: 609-622.
15. Cascio, C, V.V.K. Prasad, Y.Y. Lin, S. Lieberman and V. Papadopoulos (1998) Detection of P450c17-independent pathways for dehydroepiandrosterone (DHEA) biosynthesis in brain glial tumor cells. Proc. Natl. Acad Sci. USA 95: 2862-2867.
16. Brown, R.C., C. Cascio and V. Papadopoulos (2000) Neurosteroid biosynthesis in cell lines from human brain: regulation of dehydroepiandrosterone formation by oxidative stress and β-amyloid peptide. J. Neurochem. 74: nnnnn.
17. Markesbery, W. M. (1997) Oxidative stress hypothesis in Alzheimer's disease. Free Rad. Biol. Med. 23: 134-147.
18. Joachim, C.L. and D.J. Selkoe (1992) The seminal role of β-amyloid in the pathogenesis of Alzheimer disease. Alzheimer Dis. Assoc. Disord. 6: 7-34.
19. Nunomura, A. et al. (1999) RNA oxidation is a prominent feature of vulnerable neurons in Alzheimer's disease. J. Neuroscience 19: 1959-1964. Aβ oxidative damage to neurons.
20. Mecocci, P., U. MacGarvey and M. Beal (1994) Oxidative damage to mitochondrial DNA is increased in Alzheimer's disease. Ann. Neurol. 36: 747-751.
21. Subbararo, K.V., J.S. Richardson and L.C. Ang (1990) Autopsy samples of Alzheimer's cortex show increased peroxidation in vitro. J Neurochem. 55: 342-345.
22. Huang, X. et al. (1999) The Aβ peptide of Alzheimer's disease directly produces hydrogen peroxide through metal ion production. Biochemistry 38: 7609-7616.
23. Guazzo, E.P., P.J. Kirkpatrick, I.M. Goodyear, H.M. Shiers and J. Herbert (1996) Cortisol, dehydroepiandrosterone (DHEA), and DHEA sulfate in the cerebrospinal fluid of man: relation to blood levels and the effects of age. J. Clin. Endo. Metab. 81: 3951-3960.
24. Wolf, O.T. et al. (1997) A single administration of dehydroepiandrosterone does not enhance memory performance in young healthy adults, but immediately reduces cortisol levels. Biol. Psychiatry 42: 845-848.
25. Wolf, O.T. et al. (1997) Effects of a two-week physiological dehydroepiandrosterone substitution on cognitive performance and well-being in healthy elderly women and men. J. Clin. Endo. Metab. 82: 2363-2367.
26. Berr, C., S. Lafont, B. Debuire, J.-F. Dartigues and E.-E. Baulieu (1996) Relationships of dehydroepiandrosterone sulfate in the elderly with functional, psychological, and mental status, and short-term mortality: a French community-based study. Proc. Natl. Acad. Sci. USA 93: 13410-13415.
27. Lambert, M.P. et al. (1998) Diffusible, nonfibrillar ligands derived from Aβ1-42 are potent central nervous system neurotoxins. Proc. Natl. Acad. Sci. USA 95: 6448-6453.
28. Prasad, V.V.K., S.R. Vagesna, M. Welch and S. Lieberman (1994) Precursors of the neurosteroids. Proc. Natl. Acad. Sci. USA 91: 3220-3223.
29. Smith, M.A. et al. (1996) Oxidative damage in Alzheimer's. Nature 382: 120-121.
30. Lacroix, C. et al. (1987) Simultaneous radioimmunoassay of progesterone, androst-4-enedione, pregnenolone, dehydroepiandrosterone and 17-hydroxypregnenolone in specific regions of human brain. J. Steroid Biochem. 28: 317-325.
31. Lanthier, A. and V.V. Patwardhan (1986) Sex steroids and 5-en-3b-hydroxysteroids in specific regions of the human brain and cranial nerves. J. Steroid Biochem. 25: 445-449.
32. Kimonides, V. G., N. H. Khatibi, C. N. Svendsen, M. V. Sofroniew and J. Herbert (1998) Dehydroepiandrosterone (DHEA) and DHEA-sulfate (DHEA-S) protect hippocampal neurons against excitatory amino acid-induced neurotoxicity. Proc. Natl. Acad. Sci. USA 95: 1852-1857.
33. Canning, D.R. et al(1993) β-amyloid of Alzheimer's disease induces reactive gliosis that inhibits axonal outgrowth. Exp. Neurol. 124: 289-298.
34. Amri H., Ogwuegbu S. O., Boujrad N., Drieu K., Papadopoulos V. (1996) In Vivo Regulation of Peripheral-Type Benzodiazepine Receptor and Glucocorticoid Synthesis by Ginko biloba Extract EGb 761 and Isolated Ginkgolides. Endocrinology,137, 5707-5718.
35. Beekman M., Ungard J. T., Gasior M., Carter R. B., Dijkstra, D., Goldberg, S. R., and Witkin, J. M. (1998) Reversal of Behavioral Effects of Pentylenetetrazol by the Neuroactive Steroid Ganaxolone. J. Pharmacol. Exp. Therap., 284, 868-877.
36. Bitran D., Hilvers R. J., Kellogg C. K. (1991) Anxiolytic effects of 3a-hydroxy-5a[b]-pregnan-20-one: endogenous metabolites of progesterone that are active at the GABAA receptor. Brain Res., 561, 157-161.
37. Bitran D., Purdy R. H., Kellogg C. K. (1993) Anxiolytic Effect of Progesterone is Associated With Increases in Cortical Allopregnanolone and GABAA Receptor Function. Pharm. Biochem. Behavior, 45, 423-428.
38. Bixo M., Andersson A., Winblad B., Purdy R. H., Backstrom T. (1997) Progesterone, 5a-pregnane-3,20-dione and 3a-hydroxy-5a-pregnane-20-one in specific regions of the human female brain in different endocrine states. Brain Res., 764,173-178.
39. Bradford, M. M. (1976) A rapid and sensitive method for the quantification of microgram quantities of protein using the principle of protein-dye binding. Anal. Biochem., 72, 248-254.
40. Britton K. T., McLeod S., Koob G. F., Hauger R. (1992) Pregnane Steroid Alphaxalone Attenuates Anxiogenic Behavioral Effects of Corticotropin Releasing Factor and Stress. Pharm. Biochem. Behavior, 41, 399-403.
41. Carter R. B., Wood P. L., Wieland S., Hawkinson J. E., Belelli D., Lambert J. J., White, H. S., Wolf, H. H., Mirsadeghi, S., Tahir, S. H., Bolger, M. B., Lan, N. C., and Gee, K. W. (1997) Characterization of the Anticonvulsant Properties of Ganaxolone (CCD 1042; 3a-Hydroxy-3b-methyl-Salpha-pregnan-20-one), a Selective, High-Affinity, Steroid Modulator of the g-Aminobutyric AcidA Receptor. J. Pharmacol. Exper. Therap., 280, 1284-1295.
42. Cascio C., Papadopoulos V. (1997) Dehydroepiandrosterone and Pregnenolone Biosynthesis in the Developing Rat Brain. Soc. Neurosci. Abstracts, 23, 1154.
43. Cornu P., Benavides J., Scatton B., Hauw J. J., Philippon J. (1992) Increase in Omega 3 (peripheral-type benzodiazepine) Binding Site Densities in Different Types of Human Brain Tumors. A Quantitative Autoradiography Study. Acta Neurochir., 119, 146-152.
44. Coyle J. T., Puttfarcken P. (1993) Oxidative Stress, Glutamate and Neurodegenerative Disorders. Science, 262, 689-695.
45. Dyrks T., Dyrks E., Hartmann R., Masters C., Beyreuther K. (1992) Amyloidogenicity of beta A4 and beta A4-bearing Amyloid Protein Precursor Fragments by Metal-Catalyzed Oxidation. J. Biol. Chem., 267, 18210-18217.
46. Edgar D. M., Seidel W. F., Gee K. W., Field, G., Xia, H., Hawkinson, J. E., Wieland, S., Carter, R. B., and Wood, P. L. (1997) CCD-3693: An Orally Bioavailable Analog of the Endogenous Neuroactive Steroid, Pregnanolone, Demonstrates Potent Sedative Hypnotic Actions in the Rat. J. Pharmacol. Exper. Therap., 282, 420-429.
47. Gasior M., Carter R. B., Goldberg S. R., Witkin J. M. (1997) Anticonvulsant and Behavioral Effects of Neuroactive Steroids Alone and in Conjunction with Diazepam. J. Pharmacol. Exper. Therap., 282, 543-553.
48. Goodman Y., Mattson M. (1994) Secreted Forms of b-Amyloid Precursor Protein Protect Hippocampal Neurons Against Amyloid-b-peptide-Induced Oxidative Injury. Exp. Neurol., 128, 1-12.
49. Guarneri P., Guarneri R., Cascio C., Pavasant P., Piccoli F., Papadopoulos V. (1994) Neurosteroidogenesis in Rat Retinas. J. Neurochem., 63, 86-96.
50. Guarneri P., Papadopoulos V., Pan B., Costa E. (1992) Regulation of pregnenolone synthesis in C6-2B glioma cells by 4'-chlordiazepam. Proc. Natl. Acad. Sci. USA, 89, 5118-5122.
51. Guo Q., Crsitakos S., Robinson N., Mattson M. (1998) Calbindin D28k Blocks the Proapoptotic Actions of Mutant Presenilin 1: Reduced Oxidative Stress and Preserved Mitochondrial Function. Proc. Natl. Acad. Sci. USA, 95, 3227-3232.
52. Guo, Q., Furukawa, K., Sopher, B., Pham, D., Xie, J., Robinson, N., Martin, G., and Mattson, M. (1996) Alzheimer's PS-1 Mutation Perturbs Calcium Homeostasis and Sensitizes PC12 Cells to Death Induced by Amyloid beta-Peptide. Neuroreport, 8, 379-383.
53. Hardwick M., Fertikh D., Culty M., Li H., Vidic B., Papadopoulos V. (1999) Peripheral-type Benzodiazepine Receptor (PBR) in Human Breast Cancer: Correlation of Breast Cancer Cell Aggressive Phenotype with PBR Expression, Nuclear Localization, and PBR-mediated Cell Proliferation and Nuclear Transport of Cholesterol. Cancer Res., 59, 831-842.
54. Harris M., Hensley K., Butterfield D., Leedle R., Carney J. (1995) Direct Evidence of Oxidative Injury produced by the Alzheimer's b-Amyloid Peptide (1-40) in Cultured Hippocampal Neurons. Exp. Neurol., 131, 193-202.
55. Hensley K.; Carney J., Mattson M., Aksenova, M., Harris, M., Wu, J., Floyd, R., and Butterfield, D. (1994) A Model for beta-Amyloid Aggregation and Neurotoxicity Based on Free Radical Generation by the Peptide: Relevance to Alzheimer Disease. Proc. Natl. Acad. Sci. USA, 91, 3270-3274.
56. Hu J., Akama K. T., Krafft G. A., Chromy B. A., Van Eldik L. J. (1998) Amyloid-b peptide activates cultured astrocytes: morphological alterations, cytokine induction and nitric oxide release. Brain Res., 785, 195-206.
57. Kimonides V. G., Khatibi N. H., Svendsen C. N., Sofroniew M. V., Hervert J. (1998) Dehydroepiandrosterone (DHEA) and DHEA-sulfate (DHEA-S) protect hippocampal neurons against excitatory amino acid-induced neurotoxicity. Proc. Natl. Acad. Sci. USA, 95, 1852-1857.
58. Klegeris A., McGeer P. L. (1997) β Amyloid Protein Enhances Macrophage Production of Oxygen Free Radicals and Glutamate. J. Neurosci. Res., 49, 229-235.
59. Klegeris A., Walker D. G., McGeer P. L. (1994) Activation of Macrophages by Alzheimer b Amyloid Peptide. Biochem. Biophys. Res. Comm., 199, 984-991.
60. LaCagnin, L. B., Levitt, M., Bergstrom, J. M., Colby, H. D. (1989) Inhibition of Adrenocortical, Mitochondrial and Microsomal Monooxygenases by SU-10', a Steroid 17a-Hydroxylase Inhibitor. J. Steroid Biochem. , 33, 599-604.
61. Lee V. M., Andrews P. W. (1986) Differentiation of NTERA-2 Clonal Human Embryonal Carcinoma Cells into Neurons Involves the Induction of All Three Neurofilament Proteins. J. Neurosci., 6, 514-521.
62. LeGoascogne C., Robel P., Gouezou M., Sananes N., Baulieu E. E., Waterman M. (1987) Neurosteroids: Cytochrome P-450scc in Rat Brain. Science, 237, 1212-1215.
63. Li H., Papadopoulos V. (1998) Peripheral-Type Benzodiazepine Receptor Function in Cholesterol Transport. Identification of a Putative Cholesterol Recognition/Interaction Amino Acid Sequence and Consensus Pattern. Endocrinology, 139, 4991-4997.
64. Lyne C., Gower D. B., Lessof M. (1974) Proceedings: Effects of aminoglutethimide and SU 10'603 on the Biosynthesis of C19 Steroids. J. Endocrinol., 61, XVI-XVII.
65. Majewska M. D., Harrison, N. L., Schwartz, R. D., Barker, J. L., Paul, S. M. (1986) Steroid Hormone Metabolites are Barbiturate-like Modulators of the GABA Receptor. Science, 232, 1004-1007.
66. McGeer E. G., Singh E. A., McGeer P. L. (1988) Peripheral-Type Benzodiazepine Binding in Alzheimer Disease. Alzheimer Dis. Assoc. Disord., 2, 331-336.
67. Mellon S. H. (1994) Neurosteroids: Biochemistry, Modes of Action and Clinical Relevance. J. Clin. Endo. Metab., 78, 1003-1008.
68. Miettinen, H., Kononen, J., Haapasalo, H., Helen, P., Sallinen, P., Harjuntausta, T., Helin, H., and Alho, H. (1995) Expression of Peripheral-type Benzodiazepine Receptor and Diazepam Binding Inhibitor in Human Astrocytomas: Relationship to Cell Proliferation. Cancer Res., 15, 2691-2695.
69. Miyaki E. (1979) Establishment of a Human Oligodendroglial Cell Line. Acta Neuropathologica, 46, 51-55.
70. Moriyama T., Kataoka H., Tsubouchi H., Koono M. (1995) Concomitant expression of hepatocyte growth factor (HGF), HGF-activator and c-met genes in human glioma cells in vitro. FEBS Letters, 372, 78-82.
71. Papadopoulos V. (1993) Peripheral-type benzodiazepine/diazepam binding inhibitor receptor: biological role in steroidogenic cell function. Endocr. Rev., 14,222-240.
72. Papadopoulos V., Amri H., Li H., Boujrad N., Vidic B., Gamier M. (1997) Targeted Disruption of the Peripheral-type Benzodiazepine Receptor Gene Inhibits Steroidogenesis in the R2C Leydig Tumor Cell Line. J. Biol. Chem., 272, 32129-32135.
73. Papadopoulos V., Guarneri P., Kreuger K. E., Guidotti A., Costa E. (1992) Pregnenolone biosynthesis in C6-2B glioma cell mitochondria: Regulation by a mitochondrial diazepam binding inhibitor receptor. Proc. Natl. Acad. Sci. USA, 89, 5113-5117.
74. Paul S. M., Purdy R. H. (1992) Neuroactive Steroids. FASEB J., 6, 2311-2322.
75. Pelletier, G., Luu-The, V., Labrie, F. (1994) Immunocytochemical localization of 5a-reductase in rat brain. Mol. Cell. Neurosci. 5, 394-399.
76. Pike C. J., Cummings B. J., Monzavi R., Cotman C. W. (1994) b-Amyloid-induced Changes in Cultured Astrocytes Parallel Reactive Astrocytosis Associated with Senile Plaques in Alzheimer's Disease. Neuroscience, 63, 517-531.
77. Pleasure S. J., Lee V. M. (1993) NTera 2 cells: A Human Cell Line which Displays Characteristics Expected of a Human Committed Neuronal Progenitor Cell. J. Neurosci. Res., 35, 585-602.
78. Rendt J., Erulkar S., Andrews P. W. (1989) Presumptive neurons derived by differentiation of a human embryonal carcinoma cell line exhibit tetrodotoxin-sensitive sodium currents and the capacity for regenerative responses. Exp. Cell Res., 180, 580-584.
79. Robel, P., Young, J., Corpechot, C., Mayo, W., Perche, F., Haug, M., Simon, H., and Baulieu, E. E. (1995) Biosynthesis and Assay of Neurosteroids in Rats and Mice: Functional Correlates. J. Steroid Biochem. Molec. Biol., 53, 355-360.
80. Salinero O., Moreno-Flores M. T., Ceballos M. L., Wandosell F. (1997) b-Amyloid Peptide Induced Cytoskeletal Reorganization in Cultured Astrocytes. J. Neurosci. Res., 47, 216-223.
81. Sanne, J. L. and Krueger, K. E. (1995) Abberent splicing of rat steroid 17a-hydroxylase transcripts. Gene 165, 327-328.
82. Smith, M. A., Rudnicka-Nawrot, M., PL, R., Praprotnik, D., Mulvihill, P., Miller, C., Sayre, L., and Perry, G. (1995) Carbonyl-related Posttranslational Modification of Neurofilament Protein in the Neurofibrillary Pathology of Alzheimer's Disease. J. Neurochem., 64, 2660-2666.
83. Smith, M. A., Taneda, S., Richey, P. L., Miyata, S., Yan, S. D., Stem, D., Sayre, L. M., Monnier, V. M., and Perry, G. (1994) Advanced Maillard reaction end products are associated with Alzheimer disease pathology. Proc. Natl. Acad. Sci. USA, 91, 5710-5714.
84. Uzunova, V., Sheline, Y., Davis, J. M., Rasmusson, A., Uzunov, D. P., Costa, E., and Guidotti, A. (1998) Increase in the cerebrospinal fluid content of neurosteroids in patients with unipolar major depression who are receiving fluoxetine or fluvoxamine. Proc. Natl. Acad. Sci. USA, 95, 3239-3244.
85. Vallee, M., Mayo, W., Darnaudery, M., Corpechot, C., Yound, J., Koehl, M., Le Moal, M., Baulieu, E.-E., Robel, P., and Simon, H. (1997) Neurosteroids: Deficient cognitive performance in aged rats depends on low pregnenolone sulfate levels in the hippocampus. Proc. Natl. Acad. Sci. USA, 94, 14856-14870.
86. Younkin, D. P., Tang, C. M., Hardy, M., Reddy, U. R., Shi, Q. Y., Pleasure, S. J., Lee, V. M., and Pleasure, D. (1993) Inducible Expression of Neuronal Glutamate Receptor Channels in the NT2 Human Cell Line. Proc. Natl. Acad. Sci. USA, 90, 2174-2178.
87. Zwain, I. H., and Yen, S. S. C. (1999) Dehydroepiandrosterone: Biosynthesis and Metabolism in the Brain. Endocrinology 240, 880-887.
88. Gutteridge (1992) Iron and oxygen radicals in the brain. Ann. Neurol. 32: S16-S21.
89. Kumar et al. (1994) Mitochondria from Alzheimer's fibroblasts show decreased uptake of calcium and increased sensitivity to free radicals. Life Sci. 54: 1855-1860.
90. Araujo D. M., Cotman C. W. (1992) b-Amyloid stimulates glial cells in vitro to produce growth factors that accumulate in senile plaques in Alzheimer's disease. Brain Res., 569, 141-145.

## Claims

1. A method of detecting oxidative stress-mediated Dehydroepiandrosterone (DHEA) formation in a test sample of blood, body fluid or tissue comprising:
(a) treating said test fluid or tissue with an agent that produces oxidative metabolites in a control fluid or tissue,
(b) treating a control fluid or tissue with said agent, and
(c) determining whether the quantities of DHEA in the test sample and in the control sample rise in response to said treatment,
wherein said agent is a β-amyloid peptide, and
whereby a lack of response in the test sample relative to the control sample indicates that oxidative stress-mediated DHEA formation occurred previously in said test sample of body fluid.

2. The method according to claim 1, wherein said body fluid or tissue is selected from the group consisting of cerebrospinal fluid, serum, hippocampus tissue, oligodendrocytes and astrocytes.

3. The method according to claim 1 or claim 2, wherein said quantities of DHEA are measured by:
(i) extracting the samples with an organic solvent,
(ii) subjecting the extracted material to HPLC chromatography, and
(iii) detecting the DHEA using radioimmunoassay.

4. The method according to claim 3, wherein said radioimmunoassay is performed using a specific antibody that does not recognize any other neurosteroids.

5. The method according to claim 1 or claim 2, wherein said quantities of DHEA are measured by gas chromatography coupled to mass spectrometry.

6. The method according to any preceding claim, wherein said samples are also tested for an increase in radical oxygen species after treatment with said agent.

7. The method according to claim 6, wherein said radical oxygen species are measured using 2,7 DCF fluorescence or another method for measuring free radicals.

8. The method according to any preceding claim, wherein the correlation between the treatment and the effect is verified by determining whether the effect is blocked by vitamin E or other antioxidants.

9. The method according to any preceding claim, wherein said samples are pretreated with SU 10603.

10. An *in vitro* method of diagnosing the existence of a neuropathologic disorder in a patient comprising measuring the quantity of a neurosteroid in the CNS of said patient, wherein said neurosteroid is DHEA or a metabolic precursor for DHEA.

11. The method according to claim 10, wherein an increased quantity of neurosteroid relative to a control indicates the development of a neuropathologic disorder.

12. The method according to claim 10, wherein a decreased quantity of the neurosteroid precursor relative to a control indicates the development of a neuropathologic disorder.

13. The method according to any of claims 10 to 12, wherein cerebrospinal fluid is used as a diagnostic tool for the CNS.

14. The method according to claim 10, wherein said quantities of DHEA are measured by a method comprising:
(i) extracting the samples with an organic solvent,
(ii) subjecting the extracted material to HPLC chromatography, and
(iii) detecting the DHEA using radioimmunoassay.

15. The method according to claim 10, wherein said quantities of precursor are measured by a method comprising:
(a) providing a test sample of blood, body fluid or tissue from said patient,
(b) treating said test fluid or tissue with an agent that produces oxidative metabolites in a control fluid or tissue,
(c) treating a control fluid or tissue with said agent, and
(d) measuring the quantities of precursor in the samples by how much DHEA is formed,
whereby less precursor in the test sample relative to the control sample indicates the existence of a neuropathologic disorder.

16. The method according to claim 15, wherein said agent is FeSO₄.

17. The method according to any one of claims 10 to 16, wherein said neuropathologic disorder is Alzheimer's disease.

18. An *in -vitro* method of increasing or causing DHEA synthesis by cells, comprising exposing said cells to β-amyloid peptide, wherein such exposure results in synthesis of DHEA.

19. The method according to claim 18, wherein said cells are brain cells.

20. The method according to claim 19, wherein said brain cells are selected from the group consisting of astrocytes and oligodendrocytes.

21. The method according to any of claims 18 to 20, wherein said synthesis is inhibited by Vitamin E.

## Patentansprüche

1. Verfahren zum Nachweisen einer oxidativen Stress-vermittelten Bildung von Dehydroepiandrosteron (DHEA) in einer Testprobe von Blut, Körperflüssigkeit oder Körpergewebe, umfassend:
(a) Behandeln der Testflüssigkeit oder des Testgewebes mit einem Mittel, das oxidative Metaboliten in einer Kontrollflüssigkeit oder einem Kontrollgewebe erzeugt,
(b) Behandeln einer Kontrollflüssigkeit oder eines Kontrollgewebes mit dem Mittel und
(c) Bestimmen, ob die Mengen von DHEA in der Testprobe und in der Kontrollprobe in Reaktion auf die Behandlung ansteigen,
wobei das Mittel ein β-Amyloid-Peptid ist und
wobei ein Fehlen einer Reaktion in der Testprobe bezogen auf die Kontrollprobe anzeigt, dass in der Testprobe von Körperflüssigkeit zuvor eine oxidative Stress-vermittelte DHEA-Bildung aufgetreten ist.

2. Verfahren nach Anspruch 1, wobei die Körperflüssigkeit oder das Körpergewebe ausgewählt wird aus der Gruppe bestehend aus Hirn- und Rückenmarksflüssigkeit, Serum, Hippokampusgewebe, Oligodendrozyten und Astrozyten.

3. Verfahren nach Anspruch 1 oder Anspruch 2, wobei die Mengen von DHEA gemessen werden durch:
(i) Extrahieren der Proben mit einem organischen Lösemittel,
(ii) Unterwerfen des extrahierten Materials einer HPLC-Chromatographie, und
(iii) Detektieren des DHEA unter Verwendung eines Radioimmunoassays.

4. Verfahren nach Anspruch 3, wobei der Radioimmunoassay unter Verwendung eines spezifischen Antikörpers, der jegliche anderen Neurosteroide nicht erkennt, ausgeführt wird.

5. Verfahren nach Anspruch 1 oder Anspruch 2, wobei die Mengen von DHEA durch eine mit Massenspektrometrie gekoppelte Gaschromatographie gemessen werden.

6. Verfahren nach einem der vorangegangenen Ansprüche, wobei die Proben auch auf einen Anstieg von radikalischen Sauerstoffspezies nach einer Behandlung mit dem Mittel getestet werden.

7. Verfahren nach Anspruch 6, wobei die radikalischen Sauerstoffspezies unter Verwendung von 2,7-DCF-Fluoreszenz oder eines anderen Verfahrens zum Messen von freien Radikalen gemessen werden.

8. Verfahren nach einem der vorangegangenen Ansprüche, wobei die Korrelation zwischen der Behandlung und der Wirkung verifiziert wird, indem bestimmt wird, ob die Wirkung durch Vitamin E oder andere Antioxidationsmittel blockiert wird.

9. Verfahren nach einem der vorangegangenen Ansprüche, wobei die Proben mit SU 10603 vorbehandelt werden.

10. *In vitro*-Verfahren zum Diagnostizieren des Vorliegens einer neuropathologischen Störung oder Erkrankung bei einem Patienten, umfassend, die Menge eines Neurosteroids in dem ZNS des Patienten zu messen, wobei das Neurosteroid DHEA oder eine Stoffwechselvorstufe für DHEA ist.

11. Verfahren nach Anspruch 10, wobei eine erhöhte Menge von Neurosteroid bezogen auf eine Kontrolle die Entwicklung einer neuropathologischen Störung oder Erkrankung anzeigt.

12. Verfahren nach Anspruch 10, wobei eine verringerte Menge der Neurosteroid-Vorstufe bezogen auf eine Kontrolle die Entwicklung einer neuropathologischen Störung oder Erkrankung anzeigt.

13. Verfahren nach einem der Ansprüche 10 bis 12, wobei Him- bzw. Rückenmarksflüssigkeit als ein diagnostisches Hilfsmittel für das ZNS verwendet wird.

14. Verfahren nach Anspruch 10, wobei die Mengen von DHEA gemessen werden durch ein Verfahren, umfassend:
(i) Extrahieren der Proben mit einem organischen Lösemittel,
(ii) Unterwerfen des extrahierten Materials einer HPLC-Chromatographie, und
(iii) Detektieren des DHEA unter Verwendung eines Radioimmunoassays.

15. Verfahren nach Anspruch 10, wobei die Mengen von Vorstufe gemessen werden durch ein Verfahren, umfassend:
(a) Bereitstellen einer Testprobe von Blut, Körperflüssigkeit oder Gewebe von dem Patienten,
(b) Behandeln der Testflüssigkeit oder des Testgewebes mit einem Mittel, das oxidative Metabolite in einer Kontrollflüssigkeit oder einem Kontrollgewebe erzeugt,
(c) Behandeln einer Kontrollflüssigkeit oder eines Kontrollgewebes mit dem Mittel und
(d) Messen der Mengen von Vorstufe in den Proben **dadurch**, wie viel DHEA gebildet wird,
wobei weniger Vorstufe in der Testprobe bezogen auf die Kontrollprobe das Vorliegen einer neuropathologischen Störung oder Erkrankung anzeigt.

16. Verfahren nach Anspruch 15, wobei das Mittel FeSO₄ ist.

17. Verfahren nach einem der Ansprüche 10 bis 16, wobei die neuropathologische Erkrankung die Alzheimer-Krankheit ist.

18. *In vitro*-Verfahren zum Verstärken oder Hervorrufen einer DHEA-Synthese durch Zellen, welches umfasst, die Zellen β-Amyloid-Peptid auszusetzen, wobei eine solche Exposition zu einer Synthese von DHEA führt.

19. Verfahren nach Anspruch 18, wobei die Zellen Hirnzellen sind.

20. Verfahren nach Anspruch 19, wobei die Hirnzellen aus der Gruppe bestehend aus Astrozyten und Oligodendrozyten ausgewählt werden.

21. Verfahren nach einem der Ansprüche 18 bis 20, wobei die Synthese durch Vitamin E gehemmt wird.

## Revendications

1. Méthode pour détecter la formation de déhydroépi-androstérone (DHEA) à médiation par le stress oxydatif dans un échantillon d'essai constitué de sang, d'un fluide corporel ou d'un tissu, comprenant les étapes consistant à :
(a) traiter ledit fluide ou tissu d'essai avec un agent qui produit des métabolites oxydatifs dans un fluide ou tissu témoin ;
(b) traiter un fluide ou tissu témoin avec ledit agent ; et
(c) déterminer si les quantités de DHEA dans l'échantillon d'essai et dans l'échantillon témoin augmentent en réponse audit traitement,
dans laquelle ledit agent est un peptide ß-amyloïde, et
une absence de réponse dans l'échantillon d'essai par rapport à l'échantillon témoin indiquant que la formation de DHEA à médiation par le stress oxydatif s'est produite auparavant dans ledit échantillon d'essai de fluide corporel.

2. Méthode suivant la revendication 1, dans laquelle ledit fluide ou tissu corporel est choisi dans le groupe consistant en le liquide céphalo-rachidien, le sérum, le tissu de l'hippocampe, des oligodendrocytes et des astrocytes.

3. Méthode suivant la revendication 1 ou la revendication 2, dans laquelle lesdites quantités de DHEA sont mesurées :
(i) en soumettant les échantillons à une extraction avec un solvant organique,
(ii) en soumettant la substance extraite à une chromatographie CLHP, et
(iii) en détectant la DHEA par analyse radio-immunologique.

4. Méthode suivant la revendication 3, dans laquelle ladite analyse radio-immunologique est effectuée en utilisant un anticorps spécifique qui ne reconnaît pas de quelconques autres neurostéroïdes.

5. Méthode suivant la revendication 1 ou la revendication 2, dans laquelle lesdites quantités de DHEA sont mesurées par chromatographie en phase gazeuse couplée avec la spectrométrie de masse.

6. Méthode suivant l'une quelconque des revendications précédentes, dans laquelle lesdits échantillons sont également testés pour une augmentation des entités à radicaux oxygène après traitement avec ledit agent.

7. Méthode suivant la revendication 6, dans laquelle lesdites entités à radicaux oxygène sont dosées en utilisant la fluorescence du 2,7-DCF ou une autre méthode pour doser les radicaux libres.

8. Méthode suivant l'une quelconque des revendications précédentes, dans laquelle la corrélation entre le traitement et l'effet est vérifiée en déterminant si l'effet est bloqué par la vitamine E ou d'autres antioxydants.

9. Méthode suivant l'une quelconque des revendications précédentes, dans laquelle lesdits échantillons sont prétraités avec du SU 10603.

10. Méthode *in vitro* pour diagnostiquer l'existence d'un trouble neuropathologique chez un patient, comprenant la mesure de la quantité d'un neurostéroïde dans le SNC dudit patient, ledit neurostéroïde étant la DHEA ou un précurseur métabolique de la DHEA.

11. Méthode suivant la revendication 10, dans laquelle une quantité accrue de neurostéroïde par rapport à un témoin indique le développement d'un trouble neuropathologique.

12. Méthode suivant la revendication 10, dans laquelle une quantité réduite du précurseur de neurostéroïde par rapport à un témoin indique le développement d'un trouble neuropathologique.

13. Méthode suivant l'une quelconque des revendications 10 à 12, dans laquelle le liquide céphalo-rachidien est utilisé comme outil de diagnostic pour le SNC.

14. Méthode suivant la revendication 10, dans laquelle lesdites quantités de DHEA sont mesurées par une méthode comprenant les étapes consistant à :
(i) soumettre les échantillons à une extraction avec un solvant organique,
(ii) soumettre la substance extraite à une chromatographie CLHP, et
(iii) détecter la DHEA par analyse radio-immunologique.

15. Méthode suivant la revendication 10, dans laquelle lesdites quantités de précurseur sont mesurées par une méthode comprenant les étapes consistant à :
(a) fournir un échantillon d'essai constitué de sang, d'un fluide corporel ou d'un tissu provenant dudit patient,
(b) traiter ledit fluide ou tissu d'essai avec un agent qui produit des métabolites oxydatifs dans un fluide ou tissu témoin,
(c) traiter un fluide ou tissu témoin avec ledit agent, et
(d) mesurer les quantités de précurseur dans les échantillons par la quantité formée de DHEA,
une moindre quantité de précurseur dans l'échantillon d'essai par rapport à l'échantillon témoin indiquant ainsi l'existence d'un trouble neuropathologique.

16. Méthode suivant la revendication 15, dans laquelle ledit agent est FeSO₄.

17. Méthode suivant l'une quelconque des revendications 10 à 16, dans laquelle ledit trouble neuropathologique est la maladie d'Alzheimer.

18. Méthode *in vitro* pour augmenter ou provoquer la synthèse de DHEA par des cellules, comprenant l'exposition desdites cellules au peptide ß-amyloïde, dans laquelle cette exposition entraîne une synthèse de DHEA.

19. Méthode suivant la revendication 18, dans laquelle lesdites cellules sont des cellules cérébrales.

20. Méthode suivant la revendication 19, dans laquelle lesdites cellules cérébrales sont choisies dans le groupe consistant en les astrocytes et les oligodendrocytes.

21. Méthode suivant l'une quelconque des revendications 18 à 20, dans laquelle ladite synthèse est inhibée par la vitamine E.
